(19) Europäisches Patentamt
European Patent Office
Office européen des brevets

(11) EP 2 527 323 A1

(12) EUROPEAN PATENT APPLICATION

(43) Date of publication:
**28.11.2012 Bulletin 2012/48**

(21) Application number: **11382164.9**

(22) Date of filing: **24.05.2011**

(51) Int Cl.:
***C07C 333/10*** *(2006.01)* ***A61K 31/325*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **NOSCIRA, S.A.**
**28760 Tres Cantos, Madrid (ES)**

(72) Inventors:
- **Palomo Nicolau, Francisco**
**28760 Tres Cantos - Madrid (ES)**
- **Alonso Gordillo, Diana**
**28760 Tres Cantos - Madrid (ES)**
- **Alonso Cascón, Mercedes**
**28760 Tres Cantos - Madrid (ES)**
- **Villasante Prieto , Javier**
**28760 Tres Cantos - Madrid (ES)**

(74) Representative: **ABG Patentes, S.L.**
**Avenida de Burgos, 16D**
**Edificio Euromor**
**28036 Madrid (ES)**

# (54) Urea carbonyl disulfide derivatives and their therapeutic uses

(57) The present invention is related to compounds of Formula (I), namely novel urea carbonyl disulfide derivatives; said compounds for use as a medicament; a pharmaceutical composition comprising said compounds; said compounds for use in the treatment of certain diseases or conditions; the use of said compounds as a reactive in an *in vitro* biological assay requiring inhibition of GSK-3; the use of said compounds in the preparation of a medicament for the treatment of certain diseases or conditions; and a method of treating a disease, comprising administering said compounds.

EP 2 527 323 A1

## Description

## FIELD OF THE INVENTION

The present invention is related to a new family of compounds, namely urea carbonyl disulfide derivatives of Formula (I); said compounds for use as a medicament; a pharmaceutical composition comprising said compounds; said compounds for use in the treatment of certain diseases or conditions; the use of said compounds as a reactive in an *in vitro* biological assay requiring inhibition of GSK-3; the use of said compounds in the preparation of a medicament for the treatment of certain diseases or conditions; and a method of treating a disease, comprising administering said compounds.

## BACKGROUND OF THE INVENTION

### *Glycogen synthase kinase-3 (GSK-3)*

Glycogen synthase kinase-3 (GSK-3) is a serine/threonine protein kinase comprised of $\alpha$ and $\beta$ isoforms that are each encoded by distinct genes (Chemistry & Biology, 2000, 7(10), 793-803. *Selective small molecule inhibitors of glycogen synthase kinase-3 modulate glycogen metabolism and gene transcription.* Coghlan et al.; Curr. Opinion Genetics Dev., 2000, 10(5), 508-514. *GSK3, a master switch regulating cell-fate specification and tumorigenesis. Kim, L. & Kimmel, A.R.*). GSK-3 plays critical roles in development, metabolic homeostasis, neuronal growth and differentiation, cell polarity, cell fate and in modulation of apoptotic potential.

### *Pathologies related to Glycogen synthase kinase-3 (GSK-3)*

Dysregulation (usually increase) of GSK-3 activity is believed to play a role in different and important disorders like neurodegenerative disorders [Physiol. Rev., 2004, 84, 361-84. *Role of tau protein in both physiological and pathological conditions. Ávila, J. et al.*], cardiovascular disease [Circ Res., 2009, 104(11), 1240-52*; Role of glycogen synthase kinase-3beta in cardioprotection.* Juhaszova M. et al.; Circ J., 2009, 73(7), 1184-92*. GSK-3beta, a therapeutic target for cardiomyocyte protection. Miura T.* & *Miki T.*], diabetes [Trends. Mol. Med., 2002, 8, 126-32. Glycogen synthase kinase 3: an emerging therapeutic target. Eldar-Finkelman, H.] or viral infections [Virus Res., 2008, 132, 160-73. Residues in human respiratory syncytial virus P protein that are essential for its activity on RNA viral synthesis. Asenjo, A. *et al.*].

Regarding neurodegenerative disorders and other CNS pathologies, GSK-3 dysregulation has been related to Alzheimer's disease [Brain Res Bull., 2009, 80(4-5), 248-50. The role of GSK3 in Alzheimer disease. Hernández F. et a*l*.], Parkinson's disease [Neuroscience Letters 2009, 449(2), 103-107. Glycogen synthase kinase-3beta is associated with Parkinson's disease. Masahiro N. & Hideaki H.], frontotemporal dementia [Arch. Neurol., 2008, 65, 1368-74. Association of GSK3B with Alzheimer disease and frontotemporal dementia.. Schaffer, B. *et al.*], Pick's disease, progressive supranuclear palsy, subacute sclerosing panencephalitis, postencephalitic parkinsonism, dementia pugilistica, guam parkinsonism-dementia complex, corticobasal degeneration, argyrophilic grain disease, familial frontotemporal dementia and parkinsonism linked to chromosome 17 due to mutations in the tau gene (FTDP-17-tau) and other tauopathies [Brain Research Reviews, 2000, 33, 95-130*. Tau protein isoforms, phosphorylation and role in neurodegenerative disorders. Buée, L. et al*.], AIDS associated dementia [J Neuroimmune Pharmacol., 2007, 2(1), 93-96. Glycogen synthase kinase 3 beta (GSK-3 beta) as a therapeutic target in neuroAIDS. Dewhurst S. et a*l*.], Huntington's disease [J Biol Chem., 2002, 277(37), 33791-8. Glycogen synthase kinase-3beta inhibitors prevent cellular polyglutamine toxicity caused by the Huntington's disease mutation. Carmichael J. *et al.*], Lewy body disease *[*Neuropathology., 2003, 23(3), 199-202. Glycogen synthase kinase-3beta phosphorylates synphilin-1 in vitro. Tanji K. *et al.],* bipolar disorder [Neurosci Biobehav Rev., 2007, 31 (6), 920-931; GSK-3 is a viable potential target for therapeutic intervention in bipolar disorder. Roew M.K. *et al.;* Bipolar Disord., 2002, 4(2), 137-144. Glycogen Synthase Kinase-3$\beta$, mood stabilizers, and neuroprotection. Li X. *et al.],* depression [J Pharmacol Sci., 2009, 110(1), 14-28. Lithium and neuropsychiatric therapeutics: neuroplasticity via glycogen synthase kinase-3beta, beta-catenin, and neurotrophin cascades. Wada A.], schizophrenia [Drug News Perspect., 2007, 20(7), 437-45. The role of glycogen synthase kinase-3beta in schizophrenia. Koros E. & Domer-Ciossek C.*;* Trends Neurosci., 2007, 30(4), 142-9. Schizophrenia as a GSK-3 dysregulation disorder. Lovestone S. *et al*.], epilepsy [J Neurochem., 1999, 72(3), 1327-30. The mood-stabilizing agent valproate inhibits the activity of glycogen synthase kinase-3. Chen G. *et al.*], mood disorders [Curr Drug Targets., 2006, 7(11), 1421-34. Glycogen synthase kinase-3 (GSK3) in psychiatric diseases and therapeutic interventions. Jope R. S. & Roh M.S.], autism [Proc Natl Acad Sci U S A., 2008, 105(4), 1333-8. Role of GSK3 beta in behavioral abnormalities induced by serotonin deficiency. Beaulieu J.M. *et al.*], attention deficit hyperactivity disorder [Proc Natl Acad Sci U S A., 2004, 101(14), 5099-104. Lithium antagonizes dopamine-dependent behaviors mediated by an AKT/glycogen synthase kinase 3 signaling cascade. Beaulieu J.M. *et al.*], Down's syndrome [FASEB J., 2008, 22(9), 3224-33. Overexpression of Dyrk1A contributes to neurofibrillary degeneration in Down syndrome. Liu F. *et al.*], diseases associated with ischemia/reperfusion and shock *[*Shock., 2007, 27(2), 113-23. Glycogen synthase kinase 3beta as a target for the therapy of shock and inflammation. Dugo L. *et al.*], brain injury [Neurol Res., 2001, 23(6), 588-92. Different expression of glycogen synthase kinase-3beta between young and old rat brains after transient middle cerebral artery occlusion. Sasaki C. *et al.*], multiple sclerosis [Trends Immunol., 2010, 31(1), 24-31. Innate and adaptive immune responses regulated by glycogen synthase kinase-3 (GSK3). Beurel E. *et al.]* and other autoimmune and inflammatory diseases afflicting the CNS [J. Immunol., 2008, 181(1), 338-45. Lithium prevents and ameliorates experimental autoimmune encephalomyelitis. De Sarno P. *et al.*], spinocerebellar ataxia type 1 [PLoS Med, 2007, 4(5), 836-847. Lithium therapy improves neurological function and hippocampal dendritic arborization in a spinocerebellar ataxia type 1 mouse model. Watase K. *et al.],* cerebral bleeding for example, due to solitary cerebral amyloid angiopathy *[*Neuroscience., 2008, 153(2), 414-27. Accumulation of beta-amyloid in the brain microvessels accompanies increased hyperphosphorylated tau proteins following microsphere embolism in aged rats. Han F. *et al.]* or amyotrophic lateral sclerosis *[*Brain Res., 2008, 1196, 131-139. Upregulation of GSK3β expression in frontal and temporal cortex in ALS with cognitive impairment (ALSci). Yang W *et al.].*

In addition to its possible relevance to prevent neurodegeneration, GSK3 inhibitors may also be useful to foster other forms of neuronal repair, including axon regeneration [J. Neurosci., 2008, 28, 8914-28. Inactivation of glycogen synthase kinase 3 promotes axonal growth and recovery in the CNS. Dill, J. *et al.*].

During the last few years, GSK-3 has been identified as a regulator of many components of the immune system, suggesting it might be a plausible therapeutic target in inflammatory and autoimmune diseases, such as chronic inflammatory diseases including rheumatoid arthritis, inflammatory bowel disease and psoriasis [Eur J Biochem., 2001, 268 (19), 5001-10. The role of protein phosphorylation in human health and disease. Cohen P.], arthritis [Clin. Immunol., 2006, 120, 57-67. Glycogen synthase kinase-3b inhibition attenuates the degree of arthritis caused by type II collagen in the mouse. Cuzzocrea, S. *et al.*], peritonitis [Immunity, 2006, 24, 563-574. IFN-g suppresses IL-10 production and synergizes with TLR2 by regulating GSK3 and CREB/AP-1 proteins. Hu, X. *et al.],* systemic inflammation, renal dysfunction and hepatotoxicity in endotoxemia [Crit. Care Med., 2005, 33, 1903-1912. GSK-3b inhibitors attenuate the organ injury/dysfunction caused by endotoxemia in the rat. Dugo, L. *et al.*], asthma [Am J Physiol Lung *Ce// Mol* Physiol., 2009, 296(2), L176-84. Airway smooth muscle hyperplasia and hypertrophy correlate with glycogen synthase kinase-3(beta) phosphorylation in a mouse model of asthma. Bentley J.K. *et al.*], sepsis [J. Biochem. Cell. Biol., 2005, 37, 2226-2238. GSK-3b inhibitors reduce protein degradation in muscles from septic rats and in dexamethasone treated myotubes. Int. Evenson, A.R. *et al.*], colitis [Br. J. Pharmacol., 2006, 147, 575-582. Reduction of experimental colitis in the rat by inhibitors of glycogen synthase kinase-3b. Whittle, B.J. *et al.],* inflammation-induced organ injury caused by hemorrhage and resuscitation *[*Shock, 2006, 25, 485-491. Glycogen synthase kinase-3b inhibitors protect against the organ injury and dysfunction caused by hemorrhage and resuscitation. Dugo, L. *et al.],* inflammatory injury in chronic renal allograft disease [Am J Transplant., 2008, 8(9), 1852-63. Glycogen synthase kinase 3beta: a novel marker and modulator of inflammatory injury in chronic renal allograft disease. Gong R. *et al.*] or lupus [Int. J. Immunopharmacol., 1995, 17, 581-592. Lithium chloride enhances survival of NZBIW lupus mice: influence of melatonin and timing of treatment. Lenz, S.P. *et al.*].

Among cardiovascular disorders related to GSK-3 are heart disease [Circ. Res., 2002, 90, 1055-63. Glycogen synthase kinase-3beta: a novel regulator of cardiac hypertrophy and development. Hardt, S.E. & Sadoshima, J.], atherosclerosis [Am J Pathol., 2009, 174(1), 330-42. Valproate attenuates accelerated atherosclerosis in hyperglycemic apoE-deficient mice: evidence in support of a role for endoplasmic reticulum stress and glycogen synthase kinase-3 in lesion development and hepatic steatosis. Bowes A.J. *et al.*], hypertension [J. Clin. Invest., 2002, 109(3), 373-381. Fas receptor signaling inhibits glycogen synthase kinase 3β and induces cardiac hypertrophy following pressure overload. Badorff C. *et al.*], restenosis [Cardiovasc Res., 2010, Epub. Delayed Re-endothelialization with Rapamycin-coated Stents is Rescued by the Addition of a Glycogen Synthase Kinase 3 Beta Inhibitor. Ma X. *et al.]* or leukopenia [Gallicchio, V. S. (1991) in Lithium and the Cell, ed. Birch, N. J. (Academic, San Diego), pp. 185-198.].

Additional pathologies associated with GSK-3 are metabolic syndrome X *[*Curr Pharm Des., 2004, 10(10), 1105-37. Discovery and development of GSK3 inhibitors for the treatment of type 2 diabetes. Wagman A.S. *et al.],* hair loss *[*J Clin Invest., 2010, 120(2), 446-56. Neural Wiskott-Aldrich syndrome protein modulates Wnt signaling and is required for hair follicle cycling in mice. Lyubimova A. *et al.],* severe acute respiratory syndrome coronavirus *[*J Biol Chem., 2009, 284(8), 5229-39. Glycogen synthase kinase-3 regulates the phosphorylation of severe acute respiratory syndrome corona virus nucleocapsid protein and viral replication. Wu C.H. *et al.],* cocaine addiction [J Neurochem., 2009, 111(6), 1357-68. Glycogen synthase kinase 3beta in the nucleus accumbens core mediates cocaine-induced behavioral sensitization. Xu C.M. *et al.*], bone loss [Life Sci., 2009, 85(19-20), 685-92. Inhibition of glycogen synthase kinase-3beta attenuates glucocorticoid-induced bone loss. Wang F.S. *et al.*], fragile X syndrome (FXS) [Biochem Pharmacol., 2010, 79(4), 632-46. Lithium ameliorates altered glycogen synthase kinase-3 and behavior in a mouse model of fragile X syndrome. Yuskaitis C.J. *et al.*] or glaucoma [J Clin Invest., 2008, 118(3), 1056-64. Increased expression of the WNT antagonist sFRP-1 in glaucoma elevates intraocular pressure. Wang W.H. *et al.*].

### *GSK-3 inhibitors*

For a further review of GSK-3 inhibitors and their use as potential treatments for these pathologies, please reference to Nature Reviews, 2004, 3, 479-487. GSK3 inhibitors: development end therapeutic potential. Cohen, P. & Goedert, M.*;* Mini-Reviews in Medicinal Chemistry, 2009, 9(9), 1024-1029. GSK3 Inhibitors and Disease. Hernández, F. *et al.;* Curr. Opin. Drug Discov. Develop., 2008, 11(4), 533-543. Glycogen synthase kinase-3 (GSK-3) inhibitors reach the clinic. Medina, M. & Castro, A.*;* John Wiley & Sons, Inc., 2006. Glycogen Synthase Kinase 3 (GSK-3) and its inhibitors. Chapter 14. Eds: Martinez, A., Castro, A. & Medina, M.

Several GSK-3 inhibitors like indirubines [J. Biol. Chem., 2001, 276, 251-60. Indirubins inhibit glycogen synthase kinase-3 beta and CDK5/p25, two protein kinases involved in abnormal tau phosphorylation in Alzheimer's disease. A property common to most cyclin-dependent kinase inhibitors?. Leclerc, S. *et al.*], maleimides [Bioorg. Med. Chem. Lett., 2001, 11, 635-9. 3-Anilino- 4-arylmaleimides: potent and selective inhibitors of glycogen synthase kinase-3 (GSK-3). Smith, D. *et al.],* 3-amino pyrazoles [Bioorg. Med. Chem. Lett., 2003, 13, 1581-4. 5-arylpyrazolo[3,4-b]pyridazines: potent inhibitors of glycogen synthase kinase-3 (GSK-3). Witherington, J. *et al.*], paullones *[*Eur. J. Biochem., 2000, 267, 5983-94. Paullones are potent inhibitors of glycogen synthase kinase-3beta and cyclin-dependent kinase 5/p25. Leost, M. *et al.],* thiazoles [J. Biol. Chem., 2003, 278, 45937-45. Structural insights and biological effects of glycogen synthase kinase 3-specific inhibitor AR-A014418. Bhat, R. *et al.*] or thiadiazolidinones [J. Med. Chem., 2002, 45, 1292-9. First non-ATP competitive glycogen synthase kinase 3 beta (GSK-3beta) inhibitors: thiadiazolidinones (TDZD) as potential drugs for the treatment of Alzheimer's disease. Martinez, A. *et al.].*

In view of the above, it is of great potential therapeutic interest to find further GSK-3 inhibitors, which may be useful for treating several diseases or conditions as detailed above.

### *Phosphorylation of Tau protein*

Tau is the major neuronal microtubule associated protein (MAP); the other two known MAPs in neurons are the high molecular weight MAPs, MAP1 and MAP2. Although tau protein is found in neurons, it can be expressed in glial cells, mainly in pathological conditions, and it is possible to detect tau mRNA and proteins in several peripheral tissues such as heart, kidney, lung, muscle, pancreas, testis, as well as in fibroblasts. Tau is coded by a single gene on chromosome 17 but is expressed in several molecular isoforms that are generated by alternative splicing of its mRNA. In human brain, the alternative splicing of the mRNA results in six molecular isoforms.

Tau proteins bind to spectrin and actin filaments. Through these interactions, tau proteins may allow microtubules to interconnect with other cytoskeletal components such as neurofilaments and may restrict the flexibility of the microtubules. There is also evidence that tau proteins interact with cytoplasmic organelles. Such interactions may allow for binding between microtubules and mitochondria. The tau N-terminal projection domain also permits interactions with the neural plasma membrane. Thus, tau may act as a mediator between microtubules and plasma membrane.

Tau is a phosphoprotein and its biological activity is regulated by the degree of its phosphorylation. Normal brain tau contains 2-3 moles of phosphate per mole of the protein, which appears to be optimal for its interaction with tubulin and the promotion of microtubule assembly. In addition to phosphorylation, the alternative splicing also affects the biological activity of tau. In turn, tau proteins provide the microtubule with its own identity and physical characters (rigidity, length, stability, interactive capacity with other organelles). Therefore, by regulating microtubule assembly, tau proteins have a role in modulating the functional organization of the neuron, and particularly in axonal morphology, growth, and polarity.

For a review, please see Acta Neuropathol., 2009, 118(1), 53-69*. Mechanisms of tau-induced neurodegeneration.* Iqbal, K. et al.; Brain Research Reviews, 2000, 33, 95 -130. Tau protein isoforms, phosphorylation and role in neurodegenerative disorders. Buée, L. *et al.*]

### *Tau protein and disease*

Tau protein is abnormally hyperphosphorylated in Alzheimer's disease (AD) brain and in this form, it is the major protein subunit of the paired helical filaments (PHF) and straight filaments (SF) forming neurofibrillary tangles (NFT), neuropil threads, and plaque dystrophic neurites in AD. Tau, which is phosphorylated at over 38 serine/threonine residues in AD, is a substrate for several protein kinases. Among these kinases, glycogen synthase kinase-3 (GSK-3), cyclin dependent protein kinase-5 (cdk5), protein kinase A (PKA), calcium and calmodulin-dependent protein kinase-11 (CaMKII), casein kinase-1 (CK-1), mitogen activated protein (MAP) kinase ERK 1/2, and stress-activated protein kinases (SAPKs) have been most implicated in the abnormal hyperphosphorylation of tau.

The tau polymerized into NFT is apparently inert and neither binds to tubulin nor promotes its assembly into microtubules. Furthermore, the AD cytosolic abnormally hyperphosphorylated tau (AD Ptau) does not bind to tubulin and promote microtubule assembly, but instead it inhibits assembly and disrupts microtubules. This toxic property of

the pathological tau involves the sequestration of normal tau by the diseased protein. The AD P-tau also sequesters the other two major neuronal MAPs, MAP1 A/B and MAP2.

Neurofibrillary degeneration of abnormally hyperphosphorylated tau not only occurs in AD brain but is also seen in a family of related neurodegenerative diseases, called tauopathies. In every one of these tauopathies, the neurofibrillary changes are made up of abnormally hyperphosphorylated tau and their occurrence in the neocortex is associated with dementia. In AD brain, all of the six tau isoforms are hyperphosphorylated and aggregated into PHF. In frontotemporal dementia with Parkinsonism-linked to chromosome 17 and tau pathology (FTDP-17-tau), several missense mutations in tau co-segregate with the disease [see Acta Neuropathol., 2009, 118(1), 53-69. Mechanisms of tau-induced neurodegeneration. Iqbal, K. *et al.].*

Among these diseases characterized by abnormal hyperphosphorylation of tau are Parkinson's disease-related dementia [Mov Disord., 2009, 15, 24(15), 2203-10. Cerebrospinal tau, phospho-tau, and beta-amyloid and neuropsychological functions in Parkinson's disease. Compta, Y. *et al.*], AIDS associated dementia [Neurology, 2005, 65(9), 1490-2. CSF amyloid beta42 and tau levels correlate with AIDS dementia complex. Brew, B.J. *et al.*], Down syndrome [Neuropathol. Appl. Neurobiol., 1984, 10, 185-207. Alzheimer's presenile dementia, senile dementia of Alzheimer type and Down's syndrome in middle age form an age related continuum of pathological changes, Mann, D.M.A. *et al.*], mild cognitive impairment [J Neurol Neurosurg Psychiatry, 2009, 80(9), 966-75. CSF phosphorylated tau in the diagnosis and prognosis of mild cognitive impairment and Alzheimer's disease: a meta-analysis of 51 studies. Mitchell , A.J.], Pick disease [Arch. Pathol. Lab. Med., 2006, 130, 1063-1066. Pick disease. A Brief Overview. Frederick, J.], Lewy body dementia [Int Rev Neurobiol., 2009, 84, 215-28. Lewy body dementia. Hanson, J.C. & Lippa, C.F.*],* Niemann-Pick disease [Brain, 1985, 118, 119-129 Neurofibrillary tangles in Niemann-Pick disease type C. Love, S. *et al.*], dementia with argyrophilic grains [Brain., 2008, 131(6), 1416-32. Argyrophilic grain disease. Ferrer, I. *et al.*], frontotemporal lobar degeneration, including frontotemporal dementia, progressive apraxia and progressive non-fluent aphasia [Acta Neuropathol., 2007, 114(1), 31-8. Frontotemporal lobar degeneration: clinical and pathological relationships. Snowden, J. *et al.*], frontotemporal dementia with Parkinsonism-linked to chromosome 17 and tau pathology [Nature, 1998, 393, 702-705. Association of missense and 50-splice-site mutations in tau with the inherited dementia FTDP-17. Hutton, M. *et al.*], multiple system tauopathy with presenile dementia [Brain, 2008, 131(1), 72-89. The tauopathy associated with mutation +3 in intron 10 of Tau: characterization of the MSTD family. Spina, S. *et al.*], dementia pugilistica or chronic traumatic encephalopathy (CTE) and head trauma [Acta Neuropathol., 1992, 85, 23-30. Differential distribution of neurofibrillary tangles in the cerebral cortex of dementia pugilistica and Alzheimer's disease cases. Hof, P.R. *et al.;* J Neuropathol Exp Neurol., 2009, 68(7), 709-35. Chronic traumatic encephalopathy in athletes: progressive tauopathy after repetitive head injury. McKee, A. C. *et al.],* progressive supranuclear palsy [Lancet Neurol., 2009, 8(3), 270-9. Progressive supranuclear palsy: clinicopathological concepts and diagnostic challenges. Williams, D.R. & Lees, A.J.], postencephalitic parkinsonism [Acta Neuropathol., 2009, 118(3), 371-9. Absence of alpha-synuclein pathology in postencephalitic parkinsonism. Jellinger, K.A.], subacute sclerosing panencephalitis [Brain Dev., 2010, 32(6), 467-71. Tau proteins in the cerebrospinal fluid of patients with subacute sclerosing panencephalitis. Yuksel, D. *et al.*], amyotrophic lateral sclerosis/parkinsonism-dementia complex of Guam [Arch. Neurol., 1966, 15, 35-51. Amyotrophic lateral sclerosis and Parkinsonism-dementia complex on Guam. Further pathologic studies. Hirano, A. *et al.*], corticobasal degeneration [Psychol NeuroPsychiatr Vieil, 2009, 7(2), 91-100. Corticobasal degeneration: clinical and neuropsychological profile. Felician, O. *et al.],* pallido-ponto-nigral degeneration [Bull Acad Natl Med., 2000, 184(4), 799-809. Neurodegenerative disease associated with a mutation of codon 279 (N279K) in exon 10 of Tau protein. Delisle, M.B. *et al.],* prion disease [Biochem Soc Trans., 2010, 38(2), 545-51. Change in tau phosphorylation associated with neurodegeneration in the ME7 model of prion disease. Asuni, A.A. *et al.*], Gerstman-Sträussler-Scheinker disease [Brain Pathol., 1995, 5, 61-75. Gerstmann-Sträussler-Scheinker disease and the Indiana kindred. Ghetti, B. *et al.*], Hallervordern-Spatz disease [J Neurol Sci., 2000, 177(1), 48-59. Widespread expression of alpha-synuclein and tau immunoreactivity in Hallervorden-Spatz syndrome with protracted clinical course. Saito, Y. *et al.*], myotonic dystrophy [Acta Neuropathol., 1991, 82, 1-5. Presenile appearance of abundant Alzheimer's neurofibrillary tangles without senile plaques in the brain in myotonic dystrophy. Kiuchi, A. *et al.*], squizophrenia [Prog Neuropsychopharmacol Biol Psychiatry., 2006, 30, 30(8), 1369-80. Dysregulation of tau phosphorylation is a hypothesized point of convergence in the pathogenesis of Alzheimer's disease, frontotemporal dementia and schizophrenia with therapeutic implications. Deutsch, S.I. *et al.],* multiple sclerosis [Acta Neuropathol., 2010, 119(5), 591-600. Abnormal tau phosphorylation in primary progressive multiple sclerosis. Anderson, J.M. *et al.*], dementia and normal aging [Acta Neuropathol., 1977, 37, 111-118. Neuronal loss, neurofibrillary tangles and granulovacuolar degeneration in the hippocampus with ageing and dementia. A quantitative study. Ball, M.J.*;* J. Neurol. Sci., 1968, 7, 331-356. Observations on the brains of non-demented old people. Tomlinson, B.E. *et al.*] and glaucoma [Neurobiol Aging., Available online 7 April 2009. Tau inclusions in retinal ganglion cells of human P301 S tau transgenic mice: Effects on axonal viability. Gasparini, L. *et al.].*

For a review of pathologies wherein hyperphosphorylated tau protein is involved, please see Brain Research Reviews, 2000, 33, 95 -130. Tau protein isoforms, phosphorylation and role in neurodegenerative disorders. Buée, L. *et al.;* Eur J Neurol., 2009, 16(3), 297-309. Tauopathies with parkinsonism: clinical spectrum, neuropathologic basis,

biological markers, and treatment options. Ludolph, A.C. *et al.*

***Urea carbonyl disulfides***

Only one urea carbonyl disulfide derivative has been described so far, that is, derivatives containing the chain N-(CO)-N-(CO)-S-S, namely carbamo(dithioperoxoic) acid, [(dimethylamino)carbonyl][3-(trifluoromethyl)phenyl]-, ethyl ester:

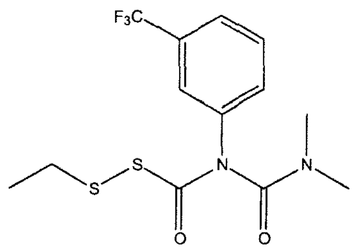

This compound has been described in US Patent Application No. US4119432, related to herbicidal compounds.

## SUMMARY OF THE INVENTION

A new family of compounds has been found, namely urea carbonyl disulfide derivatives, which exhibit an inhibition on GSK-3, and therefore have a potential therapeutic interest. Some of the compounds have also been tested for inhibition of phosphorylation of tau protein, and have shown such inhibition, thus suggesting even further therapeutic interest of the compounds of Formula (I).

Accordingly, in a first aspect, the present invention is related to a compound of Formula (I):

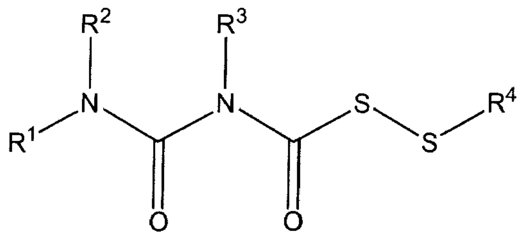

**Formula (I)**

wherein

$R^1$ is selected from optionally substituted $C_6$-$C_{14}$ aryl; $C_7$-$C_{20}$ arylalkyl; optionally substituted $C_1$-$C_6$ linear or branched alkyl; or a benzo-fused heterocyclic ring system;
$R^2$ is selected from hydrogen; or $C_1$-$C_6$ linear or branched alkyl;
$R^3$ is selected from $C_7$-$C_{20}$ arylalkyl; or optionally substituted $C_1$-$C_6$ linear or branched alkyl; optionally substituted $C_6$-$C_{10}$ aryl;
$R^4$ is selected from hydrogen, $C_3$-$C_6$ cycloalkyl; $C_7$-$C_{20}$ arylalkyl; $C_2$-$C_6$ alkenyl; optionally substituted $C_1$-$C_6$ linear or branched alkyl;

or a salt, solvate or prodrug thereof;
with the proviso that said compound is not Carbamo(dithioperoxoic) acid, [(dimethylamino)carbonyl][3-(trifluoromethyl) phenyl]-, ethyl ester:

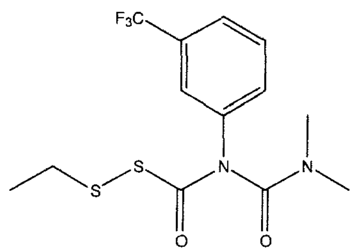

In a further aspect, the present invention is related to a compound of Formula (I) for use as a medicament:

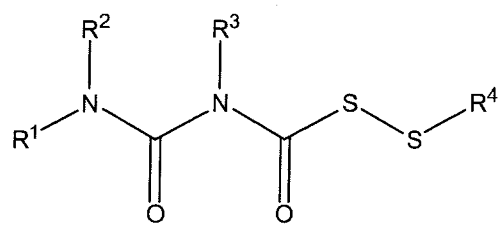

**Formula (I)**

wherein

$R^1$ is selected from optionally substituted $C_6$-$C_{14}$ aryl; $C_7$-$C_{20}$ arylalkyl; optionally substituted $C_1$-$C_6$ linear or branched alkyl; or a benzo-fused heterocyclic ring system;
$R^2$ is selected from hydrogen; or $C_1$-$C_6$ linear or branched alkyl;
$R^3$ is selected from $C_7$-$C_{20}$ arylalkyl; or optionally substituted $C_1$-$C_6$ linear or branched alkyl; optionally substituted $C_6$-$C_{14}$ aryl;
$R^4$ is selected from hydrogen, $C_3$-$C_6$ cycloalkyl; $C_7$-$C_{20}$ arylalkyl; $C_2$-$C_6$ alkenyl; optionally substituted $C_1$-$C_6$ linear or branched alkyl;

or a salt, solvate or prodrug thereof.

A further aspect of the present invention is a pharmaceutical composition comprising a compound of Formula (I) as defined above, or a salt, solvate or prodrug thereof, and at least one pharmaceutically acceptable carrier, adjuvant, and/or vehicle.

Another aspect of the present invention is a compound of Formula (I) as defined above, or a salt, solvate or prodrug thereof, for use in the treatment of a cognitive, neurodegenerative or neurological disease or condition.

A further aspect of the present invention is a compound of Formula (I) as defined above, for use in the treatment of a disease or condition selected from inflammatory and autoimmune diseases, cardiovascular disorders, and pathologies selected from metabolic syndrome X, hair loss, severe acute respiratory syndrome coronavirus, cocaine addiction, bone loss and glaucoma.

An additional aspect of the present invention is the use of a compound of Formula (I) as defined above, or a salt, solvate or prodrug thereof, as a reactive in an *in vitro* biological assay requiring inhibition of GSK-3.

In another aspect, the present invention is related to a process for obtaining a compound of Formula (II):

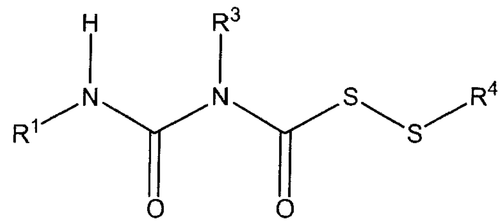

**Formula (II)**

wherein $R^1$, $R^3$ and $R^4$ have the same meanings as defined above for Formula (I), which comprises the steps of:

a) dissolving in a suitable solvent such as acetonitrile a [1,2,4]thiadiazolidine-3,5-dione of Formula (III)

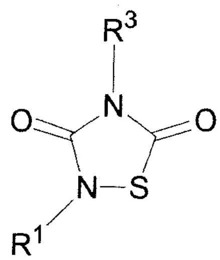

**Formula (III)**

wherein $R^1$ and $R^3$ have the same meaning as in the compound of Formula (II) as defined above;
adding a thiol derivative (e.g. as in solution) according to the desired final product: $R^4$-SH, wherein $R^4$ has the same meaning as in the compound of Formula (II);
stirring, for example, at room temperature; and

b) isolating said compound of formula (II) by known procedures such as by filtration, by preparative HPLC, or with a Flash purification system.

An additional aspect of the present invention is the use of a compound of Formula (I) as defined above, or a salt, solvate or prodrug thereof, in the preparation of a medicament for the treatment of a cognitive, neurodegenerative or neurological disease or condition.

A further aspect of the present invention is the use of a compound of Formula (I) as defined above, or a salt, solvate or prodrug thereof, in the preparation of a medicament for the treatment of a disease or condition selected from inflammatory and autoimmune diseases, cardiovascular disorders, and pathologies selected from metabolic syndrome X, hair loss, severe acute respiratory syndrome coronavirus, cocaine addiction, bone loss and glaucoma.

Another aspect of the present invention is a method of treating a cognitive, neurodegenerative or neurological disease or condition, comprising administering to a patient in need of such treatment a therapeutically effective amount of a compound of Formula (I) as defined above, or a salt, solvate or prodrug thereof.

An additional aspect of the present invention is a method of treating a disease selected from inflammatory and autoimmune diseases, cardiovascular disorders, and pathologies selected from metabolic syndrome X, hair loss, severe acute respiratory syndrome coronavirus, cocaine addiction, bone loss and glaucoma, comprising administering to a patient in need of such treatment a therapeutically effective amount of a compound of Formula (I) as defined above, or a salt, solvate or prodrug thereof.

## DEFINITIONS

In the present application the following terms have the meaning as indicated:

"Alkyl" refers to a linear or branched hydrocarbon chain radical, said chain consisting of 1 to 6 carbon atoms, preferably, 1 to 3 carbon atoms, containing no saturation, and which is attached to the rest of the molecule by a single bond, e. g., methyl, ethyl, n-propyl, i-propyl, n-butyl, t-butyl, n-pentyl, etc. These alkyl radicals may be optionally substituted by one or more substituents such as halo, hydroxy, alkoxy, carboxy, cyano, carbonyl, acyl, alkoxycarbonyl, amino, nitro, mercapto, alkylthio, acetic acid alkyl ester of formula -(CO)O-alkyl, e.g. acetic acid methyl ester -(CO)O-$CH_3$, or N-acetamide of formula -NH-(CO)-$CH_3$.

"Arylalkyl" is an alkyl as defined above, substituted by an aryl as defined herein.

"Alkenyl" means a monovalent, unbranched or branched hydrocarbon chain having one or more double bonds therein, said chain consisting of 2 to 6 carbon atoms, preferably from 2 to 4 carbon atoms. The double bond of an alkenyl group can be unconjugated or conjugated to another unsaturated group. Suitable alkenyl groups include, but are not limited to, vinyl, allyl, butenyl, pentenyl, hexenyl, butadienyl, pentadienyl, hexadienyl, 2-ethylhexenyl,2-propyl-2-butenyl, 4-(2-methyl-3-butene)-pentenyl. An alkenyl group can be unsubstituted or substituted with one or two suitable substituents.

"Aryl" refers to an aromatic ring system. According to one embodiment, aryl groups comprise 6 to 14 carbon atoms, more particularly 6 to 10, even more particularly 6 carbon atoms. According to an embodiment, aryl is a phenyl, naphthyl, indenyl, fenanthryl or anthracyl radical, preferably phenyl or naphthyl radical. Said aryl radical may be optionally substituted by one or more substituents such as hydroxy, phenoxy, mercapto, halo, alkyl, haloalkyl, phenyl, alkoxy,

haloalkyl, nitro, cyano, dialkylamino, aminoalkyl, acyl and alkoxycarbonyl.

"Benzo-fused heterocyclic ring system" refers to a phenyl ring, fused to one or two further rings, at least one of them being a heterocycle or heteroaryl, especially an oxygen containing heterocycle or heteroaryl, e.g. benzo[1,3] dioxol, benzoimidazol, benzofurane, etc.

"Cycloalkyl" refers to a stable 3-to 10-membered monocyclic or bicyclic radical which is saturated or partially saturated, the cyclic scaffold consisting solely of carbon and hydrogen atoms, preferably, 3 to 8 carbon atoms, more preferably 5, 6 or 7 carbon atoms. Unless otherwise stated specifically in the specification, the term "cycloalkyl" is meant to include cycloalkyl radicals which are optionally substituted by one or more substituents such as alkyl, halo, hydroxy, amino, cyano, nitro, alkoxy, carboxy and alkoxycarbonyl.

"Halo" or "halogen" refers to bromo, chloro, iodo or fluoro.

"Haloalkyl" refers to an alkyl radical, as defined above, that is substituted by one or more halo radicals, as defined above, e. g., trifluoromethyl, trichloromethyl, 2,2,2-trifluoroethyl,1-fluoromethyl-2-fluoroethyl, and the like.

The term "heteroaryl" means a monocyclic- or polycyclic aromatic ring comprising carbon atoms, hydrogen atoms, and one or more heteroatoms, preferably, 1 to 3 heteroatoms, independently selected from nitrogen, oxygen, and sulfur. In an embodiment of the invention, the heteroayl group has 3 to 15 members. In a particular embodiment it has 4 to 8 members. Illustrative examples of heteroaryl groups include, but are not limited to, pyridinyl, pyridazinyl, pyrimidyl, pyrazyl, triazinyl, pyrrolyl, pyrazolyl, imidazolyl, (1,2,3,)- and (1,2,4)-triazolyl, pyrazinyl, pyrimidinyl, tetrazolyl, furyl, thienyl, isoxazolyl, thiazolyl, phenyl, isoxazolyl, and oxazolyl. A heteroaryl group can be unsubstituted or substituted with one or two suitable substituents. Preferably, a heteroaryl group is a monocyclic ring, wherein the ring comprises 2 to 5 carbon atoms and 1 to 3 heteroatoms.

"Heterocycle" refers to a heterocyclyl radical. The heterocycle refers to a stable 3-to 15-membered ring which consists of carbon atoms and from one to five heteroatoms selected from the group consisting of nitrogen, oxygen, and sulfur, preferably a 4-to 8-membered ring with one or more heteroatoms, more preferably a 5- or 6-membered ring with one or more heteroatoms. For the purposes of this invention, the heterocycle may be a monocyclic, bicyclic or tricyclic ring system, which may include fused ring systems; and the nitrogen, carbon or sulfur atoms in the heterocyclyl radical may be optionally oxidised; the nitrogen atom may be optionally quaternized; and the heterocyclyl radical may be partially or fully saturated or aromatic. Examples of such heterocycles include, but are not limited to, azepines, benzimidazole, benzothiazole, furan, isothiazole, imidazole, indole, piperidine, piperazine, purine, quinoline, thiadiazole, tetrahydrofuran.

References herein to substituted groups in the compounds of the present invention refer to the specified moiety that may be substituted at one or more available positions by one or more suitable groups, e. g., halogen such as fluoro, chloro, bromo and iodo; cyano; hydroxyl; nitro; azido; alkanoyl such as a C1-6 alkanoyl group such as acyl and the like; carboxamido; alkyl groups including those groups having 1 to about 12 carbon atoms or from 1 to about 6 carbon atoms and more preferably 1-3 carbon atoms; alkenyl and alkynyl groups including groups having one or more unsaturated linkages and from 2 to about 12 carbon or from 2 to about 6 carbon atoms; alkoxy groups having one or more oxygen linkages and from 1 to about 12 carbon atoms or 1 to about 6 carbon atoms; aryloxy such as phenoxy; alkylthio groups including those moieties having one or more thioether linkages and from 1 to about 12 carbon atoms or from 1 to about 6 carbon atoms; alkylsulfinyl groups including those moieties having one or more sulfinyl linkages and from 1 to about 12 carbon atoms or from 1 to about 6 carbon atoms; alkylsulfonyl groups including those moieties having one or more sulfonyl linkages and from 1 to about 12 carbon atoms or from 1 to about 6 carbon atoms; aminoalkyl groups such as groups having one or more N atoms and from 1 to about 12 carbon atoms or from 1 to about 6 carbon atoms; carbocylic aryl having 6 or more carbons, particularly phenyl or naphthyl and aralkyl such as benzyl. Unless otherwise indicated, an optionally substituted group may have a substituent at each substitutable position of the group, and each substitution is independent of the other.

The term "pharmaceutically acceptable salts" refers to salts which, upon administration to the recipient are capable of providing (directly or indirectly) a compound as described herein. Further details are given below.

The term "prodrug" as used in this application is defined here as meaning a chemical compound having undergone a chemical derivation that yields the active compound per se after administration to a subject. Further details are given below.

The term "solvate" according to this invention is to be understood as meaning any form of the active compound according to the invention which has another molecule (most likely a polar solvent) attached to it via a non-covalent bonding.

## DETAILED DESCRIPTION OF THE INVENTION

In any of the embodiments described below, $R^4$ may be selected from $C_3$-$C_6$ cycloalkyl; $C_7$-$C_{20}$ arylalkyl; $C_2$-$C_6$ alkenyl; optionally substituted $C_1$-$C_6$ linear or branched alkyl.

In a preferred embodiment, in the compound of Formula (I) as defined above:

$R^1$ is selected from $C_6$-$C_{14}$ aryl, optionally substituted by phenoxy; $C_7$-$C_{20}$ arylalkyl; $C_1$-$C_6$ linear or branched alkyl, optionally substituted by acetic acid alkyl ester; or a benzo-fused heterocyclic ring system;
$R^2$ is selected from hydrogen; or $C_1$-$C_6$ linear or branched alkyl;
$R^3$ is selected from $C_7$-$C_{20}$ arylalkyl; or $C_1$-$C_6$ linear or branched alkyl, optionally substituted by acetic acid alkyl ester; $C_6$-$C_{14}$ aryl, optionally substituted by halogen or $C_1$-$C_6$ haloalkyl;
$R^4$ is selected from hydrogen, $C_3$-$C_6$ cycloalkyl; $C_7$-$C_{20}$ arylalkyl; $C_2$-$C_6$ alkenyl; $C_1$-$C_5$ linear or branched alkyl, optionally substituted by acetic acid alkyl ester or N-acetamide;

or a salt, solvate or prodrug thereof.

According to another particular embodiment, $R^1$ is a $C_6$-$C_{12}$ aryl or arylalkyl group, and $R^2$ is hydrogen. According to a further particular embodiment, $R^4$ is selected from the group consisting of $C_7$-$C_9$ arylalkyl (e.g. benzyl), $C_2$-$C_5$ alkyl, $C_4$-$C_6$ cycloalkyl or $C_3$-$C_5$ alkenyl, and $R^3$ is a $C_6$-$C_8$ aryl group or a $C_7$-$C_9$ arylalkyl group, preferably benzyl.

According to a further preferred embodiment thereof, in the compound of Formula (I):

$R^1$ is selected from napthyl, phenoxy-phenyl, arylmethyl, benzo[1,3]dioxol, acetic acid ethyl ester, ethyl, or methyl;
$R^2$ is selected from hydrogen, methyl, or ethyl;
$R^3$ is selected from phenylmethyl, acetic acid ethyl ester, or phenyl, optionally substituted by halogen or halomethyl;
$R^4$ is selected from hydrogen, propenyl, $C_2$-$C_5$ linear or branched alkyl, cyclopentyl, phenylethyl, phenylmethyl.

According to a further preferred embodiment thereof, in the compound of Formula (I):

$R^1$ is selected from napthyl, phenoxy-phenyl, arylmethyl, benzo[1,3]dioxol, acetic acid ethyl ester, ethyl, or methyl;
$R^2$ is selected from hydrogen, methyl, or ethyl;
$R^3$ is selected from phenylmethyl, acetic acid ethyl ester, or phenyl, optionally substituted by halogen or halomethyl;
$R^4$ is selected from propenyl, $C_2$-$C_5$ linear or branched alkyl, cyclopentyl, phenylethyl, phenylmethyl.

According to an even more preferred embodiment thereof, the compound of Formula (I) is selected from the following particular compounds:

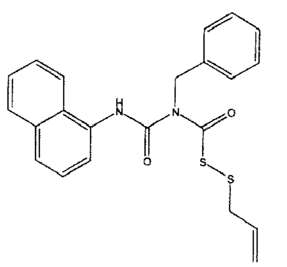
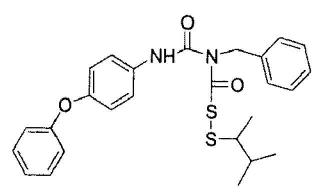

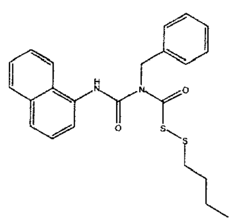
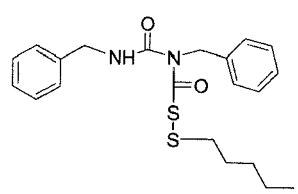

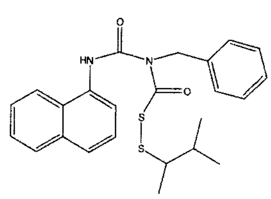
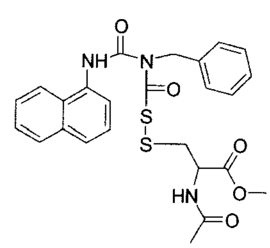

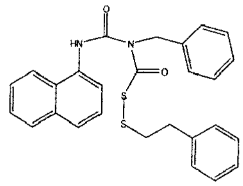

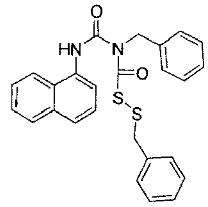

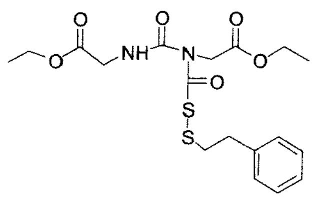

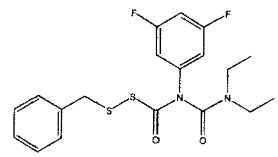

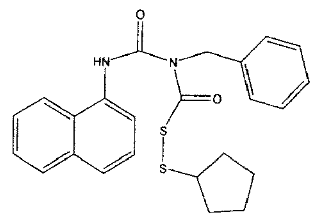

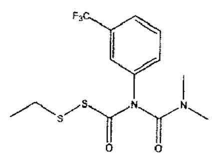

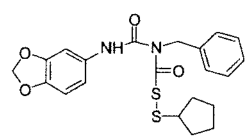

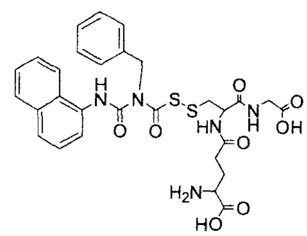

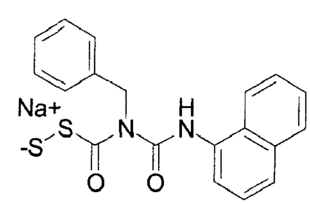

According to a preferred embodiment, the cognitive, neurodegenerative or neurological disease or condition in the above uses and methods of treatment is selected from Alzheimer's disease, Parkinson's disease, frontotemporal dementia, Pick disease, progressive supranuclear palsy, subacute sclerosing panencephalitis, postencephalitic parkinsonism, dementia pugilistica, guam parkinsonism-dementia complex, corticobasal degeneration, argyrophilic grain disease, familial frontotemporal dementia and parkinsonism linked to chromosome 17 due to mutations in the tau gene (FTDP-17-tau), AIDS associated dementia, Huntington's disease, Lewy body disease, bipolar disorder, depression, schizophrenia, epilepsy, mood disorders, autism, attention deficit hyperactivity disorder, Down's syndrome, ischemia/reperfusion, shock, brain injury, multiple sclerosis, autoimmune and inflammatory diseases afflicting the CNS, spinocerebellar ataxia type 1, cerebral bleeding due to solitary cerebral amyloid angiopathy, amyotrophic lateral sclerosis, mild cognitive impairment, Niemann-Pick disease, frontotemporal lobar degeneration, including progressive apraxia and progressive non-fluent aphasia, multiple system tauopathy with presenile dementia, chronic traumatic encephalopathy (CTE) and head trauma, pallido-ponto-nigral degeneration, prion disease, Gerstman-Sträussler-Scheinker disease, Hallervordern-Spatz disease and myotonic dystrophy.

According to a preferred embodiment, the inflammatory and autoimmune disease or condition in the above uses and methods of treatment is selected from rheumatoid arthritis, inflammatory bowel disease and psoriasis, arthritis,

peritonitis, systemic inflammation, renal dysfunction and hepatotoxicity in endotoxemia, asthma, sepsis, colitis, inflammation-induced organ injury caused by hemorrhage and resuscitation, inflammatory injury in chronic renal allograft disease and lupus.,

According to a preferred embodiment, the cardiovascular disorders in the above uses and methods of treatment are selected from heart disease, atherosclerosis, hypertension, restenosis and leukopenia.

The term "pharmaceutically acceptable salts" refers to salts which, upon administration to the recipient are capable of providing (directly or indirectly) a compound as described herein. The preparation of salts can be carried out by methods known in the art. Preferably, "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

For instance, pharmaceutically acceptable salts of compounds provided herein are synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, nonaqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, sodium, potassium, calcium, ammonium, magnesium, aluminium and lithium salts, and organic alkali salts such as, for example, ethylenediamine, ethanolamine, N,N-dialkylenethanolamine, triethanolamine, glucamine and basic aminoacids salts.

The term "prodrug" as used in this application is defined here as meaning a chemical compound having undergone a chemical derivation such as substitution or addition of a further chemical group to change (for pharmaceutical use) any of its physico-chemical properties, such as solubility or bioavailability, e.g. ester and ether derivatives of an active compound that yield the active compound per se after administration to a subject. Examples of well known methods of producing a prodrug of a given acting compound are known to those skilled in the art and can be found e.g. in Krogsgaard-Larsen et al., Textbook of Drug design and Discovery, Taylor & Francis (April 2002).

Particularly favoured prodrugs are those that increase the bioavailability of the compounds of this invention when such compounds are administered to a patient (e.g., by allowing an orally administered compound to be more readily absorbed into the blood) or which enhance delivery of the parent compound to a biological compartment (e.g., the brain or lymphatic system) relative to the parent species.

The term "solvate" according to this invention is to be understood as meaning any form of the active compound according to the invention which has another molecule (most likely a polar solvent) attached to it via a non-covalent bonding. Examples of such solvates include hydrates and alcoholates, e.g. methanolates.

The preparation of salts, solvates and prodrugs can be carried out by methods known in the art. It will be appreciated that non-pharmaceutically acceptable salts, solvates or prodrugs also fall within the scope of the invention since those may be useful in the preparation of pharmaceutically acceptable salts, solvates or prodrugs.

The compounds of the invention may be in crystalline form either as free compounds or as solvates (e.g. hydrates) and it is intended that both forms are within the scope of the present invention. Methods of solvation are generally known within the art. Suitable solvates are pharmaceutically acceptable solvates. In a particular embodiment the solvate is a hydrate.

The compounds of the present invention may exhibit tautomerism. Tautomers are one of two or more structural isomers of a compound that exist in equilibrium and are readily converted from one isomeric form to another. Common tautomeric pairs are amine-imine, amide-imidic acid, keto-enol, lactam-lactim, etc.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by a $^{13}C$- or $^{14}C$-enriched carbon or $^{15}N$-enriched nitrogen are within the scope of this invention.

Generally a "therapeutically effective amount" of the compound of the invention or a pharmaceutical composition thereof will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.

The term "treatment" or "to treat" in the context of this specification means administration of a compound or formulation according to the invention to prevent, ameliorate or eliminate the disease or one or more symptoms associated with said disease. "Treatment" also encompasses preventing, ameliorating or eliminating the physiological sequelae of the disease.

The term "ameliorate" in the context of this invention is understood as meaning any improvement on the situation of the patient treated - either subjectively (feeling of or on the patient) or objectively (measured parameters).

**Pharmaceutical compositions**

The present invention further provides pharmaceutical compositions comprising a compound of Formula (I) of the present invention, or a salt, solvate or prodrug thereof, and at least one pharmaceutically acceptable carrier, adjuvant, and/or vehicle, for administration to a patient.

The term "excipient" refers to components of a drug compound other than the active ingredient (definition obtained from the European Medicines Agency- EMA). They preferably include a "carrier, adjuvant and/or vehicle". Carriers are forms to which substances are incorporated to improve the delivery and the effectiveness of drugs. Drug carriers are used in drug-delivery systems such as the controlled-release technology to prolong *in vivo* drug actions, decrease drug metabolism, and reduce drug toxicity. Carriers are also used in designs to increase the effectiveness of drug delivery to the target sites of pharmacological actions (*U.S. National Library of Medicine. National Institutes of Health*). Adjuvant is a substance added to a drug product formulation that affects the action of the active ingredient in a predictable way. Vehicle is an excipient or a substance, preferably without therapeutic action, used as a medium to give bulk for the administration of medicines (Stedman's Medical Spellchecker, © 2006 Lippincott Williams & Wilkins). Such pharmaceutical carriers, adjuvants or vehicles can be sterile liquids, such as water and oils, including those of petroleum, animal, vegetable or synthetic origin, such as peanut oil, soybean oil, mineral oil, sesame oil and the like, excipients, disgregants, wetting agents or diluents. Suitable pharmaceutical carriers are described in "Remington's Pharmaceutical Sciences" by E.W. Martin.

According to a preferred embodiment, the pharmaceutical composition may further contain a therapeutically effective amount of one or more compounds useful for the treatment and/or prophylaxis of cognitive, neurodegenerative or neurological diseases or conditions. According to another embodiment, the pharmaceutical composition may further contain a therapeutically effective amount of one or more compounds selected from the group comprising beta secretase inhibitors or modulators including BACE1 protein inhibitors, amyloid beta-protein inhibitors, including immunoglobulins, antiamyloid monoclonal antibodies and vaccines, amyloid beta-protein precursor inhibitors, gamma secretase inhibitors or modulators, muscarinic receptor modulators, acetylcholinesterase inhibitors, butyrilcholinesterase inhibitors, Choline acetyltransferase stimulants, HMG-CoA reductase inhibitors, non-steroidal antiinflammatory agents, cyclo-oxygenase 2 inhibitors, N-methyl-D-aspartate receptor antagonists, vitamin E, nicotinic acetylcholine receptor modulators, serotonin receptor modulators, cannabinoid receptor agonists, CB1 receptor inverse agonists or CB1 receptor antagonists, AMPA receptor modulators, GABA receptor modulators, inhibitors of amyloid aggregation, glycogen synthase kinase beta inhibitors, promoters of alpha secretase activity, phosphodiesterase 9A and 10 inhibitors, type 4 cyclic nucleotide phosphodiesterase inhibitors, estrogen and cholesterol absorption inhibitors, 11-beta hydroxysteroid dehydrogenase type 1 inhibitors, adenosine receptor antagonists, adrenergic receptor modulators, advanced glycosylation end-product receptor antagonists, alpha-synuclein inhibitors, antioxidants, free radical scavengers, apolipoprotein A stimulants, apolipoprotein E agonists, apoptosis inhibitors, calcium channel modulators, sodium channel modulators, calpain inhibitors, cathepsin B inhibitors, cell-replacements including stem-cell-therapies, glial cell line-derived neurotrophic factor agonists, nerve growth factor stimulants, chelating agents, complement factor D inhibitors, cyclic AMP response element-binding protein stimulants, D amino acid oxidase inhibitors, dopamine receptor agonists and dopamine uptake inhibitors, endopeptidase inhibitors, fibroblast growth factor stimulants, G protein-coupled receptor antagonists, gene expression stimulants, glucose stimulants, metabotropic glutamate receptor modulators, histamine H3 receptor antagonists or inverse agonists, histone deacetylase inhibitors, mitochondrial-permeability-transition pore-modulators, monoamine oxidase B inhibitors, neuropeptide stimulants, neurotransmitter modulators, plasminogen activator inhibitor-1 inhibitors, protein kinase C stimulants, rho-associated kinase inhibitors, ribonucleotide reductase inhibitors, signal transduction pathway inhibitors, superoxide dismutase stimulants, tau protein modulators, tubulin polymerisation promoters, toll-like receptor agonists, transglutaminase inhibitors and Wnt protein modulators.

Examples of pharmaceutical compositions include any solid (tablets, pills, capsules, granules etc.) or liquid (solutions, suspensions or emulsions) composition for oral, topical or parenteral administration, among others.

Pharmaceutical dosage forms include but are not limited to parenteral preparations (such as injections, powders for injections, implants, etc), liquid preparations for oral use (such us syrups, solutions, suspensions, emulsions, powders and granules for suspension and for solution, oral drops, etc), oromucosal preparations (such as lozenges, sublingual and buccal tablets, oromucosal drops and sprays, etc) solid preparations for oral use (oral powders, effervescent powders, tablets -uncoated, coated, effervescent, soluble, dispersible, orodispersible, modified release, gastro-resistant,- oral lyophilisates, capsules - hard, soft, modified release, gastro-resistant-, granules - coated, effervescent, modified release, gastro-resistant-), transdermal patches, powders for inhalation, nasal preparations and rectal preparations.

In a preferred embodiment the pharmaceutical compositions are in oral form because of the convenience for the patient and the chronic character of many of the diseases to be treated. Said oral pharmaceutical compositions may

contain conventional excipients known in the art, such as:

Film coated tablet:

- Binders, such as maize starch, pregelatinised maize starch, povidone, gelatine, etc
- Diluents or fillers, such as microcrystalline cellulose, lactose, sodium phosphate, calcium phosphate dibasic dihydrate, calcium phosphate dibasic anhydrous (Emcompress, Di-tab, Di-cal-fos), etc
- Disintegrants, such as sodium croscarmellose (Acdisol, Explocel, Vivasol), sodium starch glycolate (Glycolis, Explotab, Primojel, Vivastar), cross-linked povidone, gums, etc.
- Glidants, such as talc or colloidal silica.
- Lubricants, such as magnesium stearate, stearic acid, sodium stearyl fumarate, etc
- Film-formers, such as hydroxypropylcellulose (Klucel, Metocel), Hypromellose (Metocel, Metolose, Pharmacoat), hydroxypropylmethylcellulose, etc
- Opacifiers, such as titanium dioxide.
- Colouring agents, such as sunset yellow, iron oxides, indigo carmine, erythrosine, etc
- Plasticizers, such as polyethyleneglycol, triacetin, etc

Powder for oral solution (POS) in sachet

- Acidifying agents, such as citric acid.
- Buffering agents, such as citric acid, sodium citrate
- Diluents or fillers, such as mannitol (Pearlitol), sorbitol (Neosorb, Parteck), sucrose, maltose (Advantose), etc
- Sweetening agents, such as sucralose, aspartame, accesulfame, sodium saccharine, etc
- Glidants, such as colloidal silicon dioxide (Aerosil,Cabosil,Aeroperl)
- Flavouring agents, such as strawberry flavour, lemon flavour, cola flavour, orange flavour, etc
- Thickening or stabilisers such as modified celluloses (hydroxipropylcellulose, carboxymethylcellulose sodium,...), povidones, gums, etc

Syrup

- Antimicrobial and solvent agents, such as ethanol, propyleneglycol, etc.
- Sweetening agents, such as sorbitol or sucrose
- Antimicrobial preservative, such as sodium benzoate, potassium sorbate
- Acidifying agents, such as citric acid or ascorbic acid
- Buffering agents, such as citric acid and sodium citrate, phosphates, acetic acid and sodium acetate.
- Flavouring agents, such as vainille flavour, strawberry flavour, cola flavour, peach flavour, etc
- Colouring agents, such as tartrazine, curcumin, quinoline yellow, sunset yellow, etc

Capsules

- Diluents, such as microcrystalline cellulose, lactose, calcium carbonate, calcium phosphate dibasic, calcium phosphate monobasic, calcium sulphate
- Disintegrants, such as sodium starch glycolate, cross-linked povidone.
- Lubricants, such as talc, magnesium stearate, stearic acid, sodium stearyl fumarate, polyethylenglycols, etc.

Gastrorresistant capsules

- Capsule fillers, such as microcrystalline cellulose, sugar spheres.

- Binders and film formers, such as copolymers methacrylate acid, polymeric methacrylates (Eudragit, Kollicoat)
- Plasticizers and film formers such as dibutylphthalate
- Colouring agents, such as erythrosine, sunset yellow, indigo carmine, etc
- Solvents, such as acetone, isopropyl alcohol, etc

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating. Particular examples are given below.

The pharmaceutical compositions may also be adapted for parenteral administration, such as sterile solutions, suspensions or lyophilized products in the appropriate unit dosage form. Adequate excipients can be used, such as:

- Antimicrobial preservatives, such as methylparaben, prophylparaben, etc.
- Antioxidants, such as sodium metabisulfite, propyl gallate, etc
- Stabilizing and suspending agents, such as soluble or swellable modified celluloses, e.g. carboxymethylcellulose sodium (Aquasorb, Blanose, Nymcel)
- Tonicity agents, such as sodium chloride
- Solubilizers, such as propyleneglycol or polyethyleneglycols

Particular examples are given below.

The mentioned formulations will be prepared using standard methods such as those described or referred to in the Spanish and US Pharmacopoeias and similar reference texts.

In the following, the present invention is further illustrated by examples. They should in no case be interpreted as a limitation of the scope of the invention as defined in the claims.

## EXAMPLES

## PREPARATION OF THE COMPOUNDS

### Example 1: General procedure

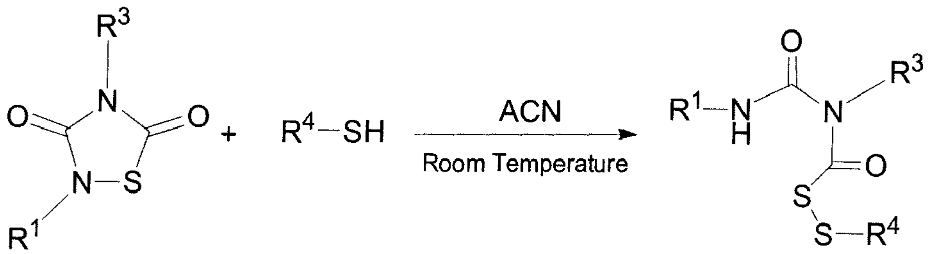


In a 100 mL round bottom flask, the corresponding [1,2,4]thiadiazolidine-3,5-dione was dissolved in 20 mL of acetonitrile. Subsequently, the corresponding thiol derivative was added to the solution and the reaction mixture was stirred overnight at room temperature. The final material was isolated either by filtration, if precipitated in the reaction mixture, or by preparative HPLC (Apparatus used: HPLC-Waters 2695-PDA2996; Column: Sunfire C18 19 x 250 mm (10 μm); Mobile phase: A: Milli-Q Water 0.1 % Formic acid; B: Acetonitrile 0.1 % Formic acid; Gradient; 17.5 mL/min; 30˚C; UV detection at 210 nm) or by Flash purification system (SP 1; Mobile phase: A: Water 0.1 % Formic acid; B: Acetonitrile 0.1 % Formic acid; Gradient)

The preparation of **[1,2,4]thiadiazolidine-3,5-dione** derivatives, used as reagents in the preparation of the compounds according to the present invention, is described, among others, in the International Patent Application WO 01/85685 and WO 05/97117, and in J. Med. Chem. 2002, 45, 1292-1299. First Non-ATP Competitive Glycogen Synthase Kinase 3 α (GSK-3α) Inhibitors: Thiadiazolidinones (TDZD) as Potential Drugs for the Treatment of Alzheimer's Disease. Martinez, A. *et al.*

The above documents are herewith incorporated by reference.

Following the above general procedure, the following compounds according to the invention were prepared:

**Table 1**

| Compound No. | |
|---|---|
| Compound 1 | 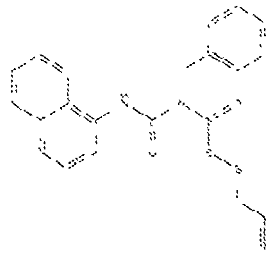 |
| Compound 2 | 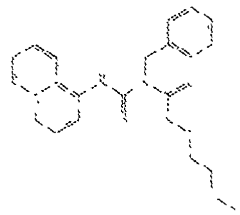 |
| Compound 3 | 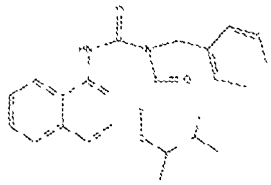 |
| Compound 4 | 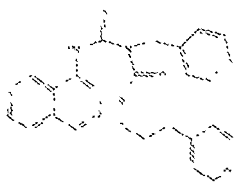 |
| Compound 5 | 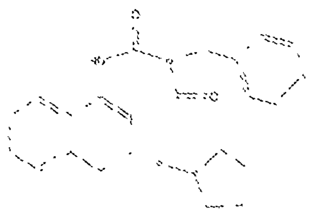 |
| Compound 6 | 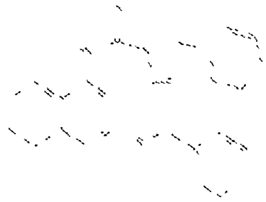 |

(continued)

| Compound No. | |
|---|---|
| Compound 7 | 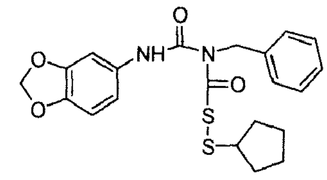 |
| Compound 8 | 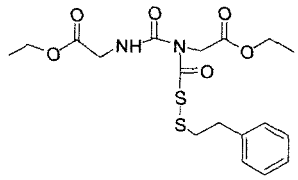 |
| Compound 9 | 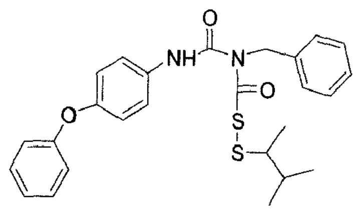 |
| Compound 10 | 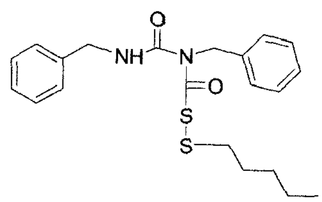 |
| Compound 11 | 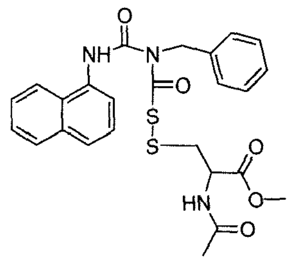 |
| Compound 14 | 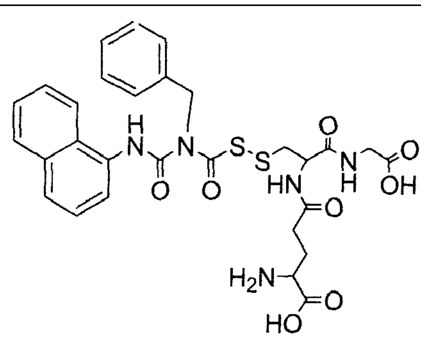 |

(continued)

| Compound No. | |
|---|---|
| Compound 15 | 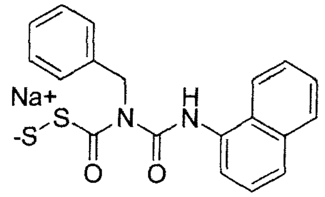 |

In the following, the particular reagents necessary for obtaining the above compounds, as well as their experimental spectral data, are indicated.

**Example 2: Preparation of Compound 1**

**1-Benzyl-1-hex-2,3-dithia-5-enoyl-3-naphthalen-1-yl-urea**

Reagents: 200 mg (0.6 mmol) 4-benzyl-2-naphthalen-1-yl-[1,2,4]thiadiazolidine-3,5-dione, 49.5 µL (0.6 mmol) 2-Propene-1-thiol.

Conditions: room temperature overnight.

Isolation: preparative HPLC

**$^1$H-NMR** ($CDCl_3$): 11.31 (1H, br), 8.14 (dd, 1H, J= 0.83, 7.62 Hz), 8.04 (dd, 1H, J= 0.57, 8.02 Hz), 7.88 (dd, 1H, J= 0.87, 9.01 Hz), 7.69 (d, 1H, J=8.27 Hz), 7.62-7.47 (m, 3H), 7.40 -7.30 (m, 5H), 5.85-5.76 (m, 1H), 5.31 (s, 2H), 5.15-5.05 (m, 2H), 3.43 (d, 2H, J= 7.39 Hz)

**$^{13}$C-NMR** ($CDCl_3$): 173.23, 151.02, 136.17, 134.03, 132.45, 131.50, 128.66, 127.71, 127.05, 126.58, 126.39, 126.04, 125.74, 125.26, 120.83, 119.94, 119.13, 47.79, 41.60.

**Example 3: Preparation of Compound 2**

**1-Benzyl-1-heptan-2,3-dithia-1-oyl-3-naphthalen-1-yl-urea**

Reagents: 200 mg (0.6 mmol) 4-benzyl-2-naphthalen-1-yl-[1,2,4]thiadiazolidine-3,5-dione, 64.2 µL (0.6 mmol) 1-Butanethiol.

Conditions: room temperature overnight.

Isolation: preparative HPLC

**$^1$H-NMR** (DMSO-$d_6$): 10.35 (1H, br), 7.95 (dd, 1H, J= 0.85, 8.13 Hz), 7.83 (dd, 1H, J= 0.57, 8.16 Hz), 7.61-7.33 (m, 9H), 5.26 (s, 2H), 2.76 (t, 2H, J= 7.20 Hz), 1.55 (q, 2H, J= 7.28 Hz), 1.35 (q, 2H, J= 7.28 Hz), 0.86 (t, 3H, J=7.29 Hz)

**$^{13}$C-NMR** (DMSO-$d_6$): 170.28, 152.85, 136.53, 133.57, 132.37, 131.50, 128.56, 128.15, 127.36, 126.54, 126.30, 126.19, 126.11, 125.50, 122.46, 122.01, 48.00, 37.40, 30.24, 20.81, 13.40

**Example 4: Preparation of Compound 3**

**1-Benzyl-1-(4,5 dimethy-hexan-2,3-dithia-1-oyl)-3-naphthalen-1-yl-urea**

Reagents: 200 mg (0.6 mmol) 4-benzyl-2-naphthalen-1-yl-[1,2,4]thiadiazolidine-3,5-dione, 62.52 mg(0.6 ml) 3-Methyl-2-Butanethiol.

Conditions: room temperature overnight.

Isolation: preparative HPLC

**$^1$H-NMR** (DMSO-$d_6$): 10.33 (1H, br), 7.96 (dd, 1H, J= 0.86, 8.15 Hz), 7.84 (dd, 1H, J= 0.57, 8.16 Hz), , 7.63-7.32 (m, 9H), 5.27 (s, 2H), 2.90 (m, 1H), 1.86 (m, 1H), 1.18 (d, 3H, J= 7.00 Hz), 0.96 (d, 3H, J=6.85 Hz), 0.93 (d, 3H, J=6.74 Hz)

**$^{13}$C-NMR** (DMSO-$d_6$): 170.49, 152.82, 136.58, 133.59, 132.39, 128.55, 128.16, 128.11, 127.34, 126.55, 126.27, 126.19, 126.11, 125.50, 122.41, 122.01, 52.76, 48.05, 37.58, 19.77, 17.87, 15.83

**Example 5: Preparation of Compound 4**

**1-Benzyl-1-(5-phenyl-pentan-2,3-dithia-1-oyl)-3-naphthalen-1-yl-urea**

Reagents: 200 mg (0.6 mmol) 4-Benzyl-2-naphthalen-1-yl-[1,2,4]thiadiazolidine-3,5-dione, 80 μL (0.6 mmol) 2-Phenylethanethiol.
Conditions: room temperature overnight.
Isolation: the precipitate is collected by filtration and washed carefully with further acetonotrile.
**$^1$H-NMR** (DMSO-$d_6$): 10.36 (1H, br), 7.97 (dd, 1H, J= 0.86, 8.17 Hz), 7.85 (dd, 1H, J= 0.57, 8.16 Hz), 7.64-7.21 (m, 14 H), 5.29 (s, 2H), 3.05(m, 2H), 2.91 (m, 2H)
**$^{13}$C-NMR** (DMSO-$d_6$): 170.20, 152.84, 139.49, 136.58, 133.58, 132.38, 128.57, 128.49, 128.25, 128.15, 128.12, 127.36, 126.55, 126.28, 126.20, 126.11, 125.50, 122.42, 122.01, 48.03, 38.80, 34.36

**Example 6: Preparation of Compound 5**

**1-Benzyl-1-(3-cyclopentyl-propion-2,3-dithia-1-oyl)-3-naphthalen-1-yl-urea**

Reagents: 200 mg (0.6 mmol) 4-Benzyl-2-naphthalen-1-yl-[1,2,4]thiadiazolidine-3,5-dione, 69 μL (0.6 mmol) Cyclopentanethiol.
Conditions: room temperature overnight.
Isolation: the precipitate is collected by filtration and washed carefully with further acetonotrile.
**$^1$H-NMR** (DMSO-$d_6$): 10.39 (1H, br), 7.97 (dd, 1H, J= 0.86, 8.16 Hz), 7.84 (dd, 1H, J= 0.57, 8.15 Hz), 7.65-7.34 (m, 9H), 5.25 (s, 2H), 3.39 (m, 1H), 1.96-1.90 (m, 2H), 1.69-1.60 (m, 2H), 1.59-1.55 (m, 4H)
**$^{13}$C-NMR** (DMSO-$d_6$): 170.40, 152.75, 136.55, 133.57, 132.38, 128.55, 128.15, 128.06, 127.34, 126.57, 126.25, 126.20, 126.11, 125.50, 122.34, 121.98, 49.32, 48.07, 32.22, 24.09

**Example 7: Preparation of Compound 6**

**1-Benzyl-3-naphthalen-1-yl-1-(4-phenyl-butyr-2,3-dithia-1-oyl)-urea**

Reagents: 200 mg (0.6 mmol) 4-Benzyl-2-naphthalen-1-yl-[1,2,4]thiadiazolidine-3,5-dione, 70 μL (0.6 mmol) Benzyl mercaptan.
Conditions: room temperature overnight.
Isolation: The precipitate is collected by filtration and washed carefully with further acetonotrile.
**$^1$H-NMR** (DMSO-$d_6$): 10.31 (1H, br), 7.94 (dd, 1H, J= 0.87, 8.14 Hz), 7.82 (dd, 1H, J= 0.78, 8.15 Hz), 7.58 -7.24 (m, 14 H), 5.25 (s, 2H), 4.02 (s, 2H)
**$^{13}$C-NMR** (DMSO-$d_6$): 169.99, 152.93, 136.67, 133.77, 132.51, 129.53, 128.78, 128.47, 128.36, 128.27, 127.58, 127.54, 127.33, 126.76, 126.47, 126.42, 126.33, 125.70, 122.56, 122.16, 48.12, 41.66

**Example 8: Preparation of Compound 7**

**3-Benzo[1,3]dioxol-5-yl-1-benzyl-1-(1-propion-2,3-dithia-3-cyclopentyl-1-yl)-urea**

Reagents: 200 mg (0.6 mmol) 2-Benzo[1,3]dioxol-5-yl-4-benzyl-[1,2,4]thiadiazolidine-3,5-dione, 65 μL (0.6 mmol) Cyclopentanethiol.
Conditions: room temperature overnight.
Isolation: Flash chromatography
**$^1$H-NMR** (DMSO-$d_6$): 10.11 (1H, br), 7.38-7.27 (m, 5 H), 7.10 (m, 1H), 6.88 (m, 2H), 6.01 (s, 2H), 5.09 (s, 2H), 1.93-1.87 (m, 2H), 1.69-1.49 (m, 7H)
**$^{13}$C-NMR** (DMSO-$d_6$): 169.27, 151.73, 147.12, 143.86, 136.53, 131.45, 128.43, 127.26, 126.69, 113.81, 107.93, 102.69, 101.12, 49.36, 48.34, 32.16, 24.06

**Example 9: Preparation of Compound 8**

**[3-Ethoxycarbonylmethyl-3-(pentan-2,3-dithia-5-phenyl-1-yl)-ureido]-acetic acid ethyl ester**

Reagents: 200 mg (0.7 mmol) (2-Ethoxycarbonylmethyl-3,5-dioxo-[1,2,4]thiadiazolidin-4-yl)-acetic acid ethyl ester, 94 μL (0.7 mmol) 2-Phenylethanethiol.

Conditions: room temperature overnight.

Isolation: Flash chromatography

**$^1$H-NMR** (DMSO-$d_6$): 10.11 (1H, br), 7.30-7.20 (m, 5 H), 4.57 (s, 2H), 4.15 (q, 2H, J= 7.10 Hz), 4.12 (q, 2H, J=7.10 Hz), 3.90 (d, 2H, J= 5.61 Hz), 2.99-2.86 (m, 4H), 1.20 (t, 3H, J=1.19 Hz)

**$^{13}$C-NMR** (DMSO-$d_6$): 169.74, 169.20, 167.53, 153.97, 139.51, 128.46, 128.27, 126.21, 60.11, 60.53, 46.44, 42.01, 38.74, 34.32, 13.96, 13.90

**Example 10: Preparation of Compound 9**

**1-Benzyl-1-(hexan-2,5-dithia-4,5-dimethyl-1-yl)-3-(4-phenoxy-phenyl)-urea**

Reagents: 200 mg (0.53 mmol) 4-Benzyl-2-(4-phenoxy-phenyl)-[1,2,4]thiadiazolidine-3,5-dione, 65 μL (0.53 mmol) 3-Methyl-butane-2-thiol.

Conditions: room temperature overnight.

Isolation: Flash chromatography

**$^1$H-NMR** (DMSO-$d_6$): 10.19 (1H, br), 7.50-7.00 (m, 14 H), 5.11 (s, 2H), 2.88-2.81 (m, 1H), 1.87-1.79 (m, 1H), 1.15 (d, 3H, J= 6.98 Hz), 0.93 (d, 3H, J=6.84 Hz), 0.90 (d, 3H, J=6.79 Hz)

**$^{13}$C-NMR** (DMSO-$d_6$): 169.34, 156.91, 152.79, 151.86, 136.57, 133.04, 129.89, 128.44, 127.26, 126.65, 123.11, 122.35, 119.18, 118.05, 52.73, 48.35, 31.48, 19.76, 17.80, 15.71

**Example 11: Preparation of Compound 10**

**1,3-Dibenzyl-1-(octan-2,3-dithia-1-oyl)-urea**

Reagents: 200 mg (0.67 mmol) 2,4-Dibenzyl-[1,2,4]thiadiazolidine-3,5-dione, 83 μL (0.67 mmol) Pentane-1-thiol.

Conditions: room temperature overnight.

Isolation: Flash chromatography

**$^1$H-NMR** (DMSO-$d_6$): 8.61 (t, 1H, J=5.68 Hz), 7.35 -7.13 (m, 10 H), 5.06 (s, 2H), 4.31 (d, 2H, J=5.72 Hz), 2.68 (t, 2H, J=7.23 Hz), 1.54-1.50 (m, 2H), 1.31-1.23 (m, 4H), 0.83 (t, 3H, J=7.07 Hz)

**$^{13}$C-NMR** (DMSO-$d_6$): 169.55, 153.99, 138.60, 136.53, 128.39, 128.12, 127.16, 126.93, 126.77, 126.28, 47.51, 43.55, 37.52, 29.79, 27.78, 21.55, 13.68

**Example 12: Preparation of Compound 11**

**2-Acetylamino-6-(1-benzyl-3-naphthalen-1-yl-ureido)-4-5-dithia-6-oxo-hexanoic acid methyl ester**

Reagents: 200 mg (0.6 mmol) 4-benzyl-2-naphthalen-1-yl-[1,2,4]thiadiazolidine-3,5-dione, 106.5 mg (0.6 mmol) N-Acetyl-L-cysteine methylester.

Conditions: room temperature overnight.

Isolation: The precipitate is collected by filtration and washed carefully with further acetonitrile.

**$^1$H-NMR** (DMSO-$d_6$): 10.22 (1H, br), 8.44 (d, 1H, J=8.45 Hz), 7.95 (d, 1H, J= 7.78 Hz), 7.84 (dd, 1H, $J_1$=6.29 Hz $J_2$=2.49 Hz), 7.54-7.35 (m, 9H), 5.29 (s, 2H), 4.57-4.51 (m, 1H), 3.62 (s, 3H), 3.15-3.01 (m, 2 H), 1.85 (s, 3H).

**$^{13}$C-NMR** (DMSO-$d_6$): 170.95, 170.70, 169.40, 155.52, 140.10, 134.98, 133.62, 128.28, 127.14, 126.72, 125.82, 125.66, 125.44, 125.35, 122.05, 121.22, 116.42, 52.17, 52.07, 51.11, 42.88, 22.21

**Example 13: Preparation of Compound 12**

Compound 13 may be prepared following the general procedure described in patent publications US4119432A and DE1219925.

**Example 14: Preparation of Compound 13**

Compound 13 may be prepared following the general procedure described in patent publications US4119432A and DE1219925.

**Example 15: Preparation of Compound 14**

**2-Amino-4-[5-(1-benzyl-3-naphthalen-1-yl-ureido)-1-(carboxymethyl-carbamoyl)-5-oxo-3,4-dithia-pentylcarbamoyl]-butyric acid**

Reagents: 0.40 mg (1.2 μmol) 4-benzyl-2-naphthalen-1-yl-[1,2,4]thiadiazolidine-3,5-dione in 1 ml of AcN and 0.36 mg (1.2 μmol) of L-Glutathione in 1 ml of $H_2O$.

Conditions: room temperature, 1 hour.

Detection: analytical HPLC (Apparatus used: HPLC-Waters 2695-PDA2996. Column: Sunfire C18 2.1 x 100 mm. Mobile phase: A: Milli-Q Water 0.1 % Formic acid B: Acetonitrile 0.1 % Formic acid. Gradient. 0.25 mL/min. 30°C. UV detection at 210 nm). In these conditions de retention time (rt) was 6.036 min. [M+1]= 642

**Example 16: Preparation of Compound 15**

**Benzyl-(N-Naphthalen-1-yl-formamide)-carbamoyl sodium dithioperoxol**

Reagents: 10.07 mg (0.03 mmol, 1 eq) of 4-benzyl-2-naphtalen-1-yl[1,2,4]tiadiazolidine-3,5-dione in 7 ml of $CH_3CN$ added over 2.51 mg (0,03 mmol, 1 eq) of sodium hydrogensulfate Hydrate in 7 ml of $H_2O$.

The resulting yellow solution was frozen with liquid nitrogen and lyophilized, obtaining 11.6 mg of a white solid (yield: 99%).

$^1$**H NMR** (400 MHz, DMSO-$d_6$) δ ppm: 13,81 (s, 1H, NH), 8.14 (dd, J = 7.67, 1.01 Hz, 1H), 8.09 (d, J = 8.47 Hz, 1H, ArH), 7.90 (dd, J = 8.26, 1.05 Hz, 1H, ArH), 7.55 (m, 3H, ArH), 7.40 (t, J = 7.94 Hz, 1H, ArH), 7.32 (dd, J = 8.03, 0.96 Hz, 2H, ArH), 7.25 (t, J = 7.58 Hz, 2H, ArH), 7.14 (t, J = 7.24 Hz, 1H, ArH), 5,62 (s, 2H, $CH_2$).

**BIOLOGICAL DATA**

**Example 17: GSK-3β assay**

The enzymatic activity of GSK3β was determined with a commercial system based on the Z'-LYTE® technology, available from Invitrogen Life Technologies (Carlsbad, CA, USA), using human recombinant GSK3β (N-terminal 6His-tagged recombinant enzyme with an H350L mutation) from Millipore (Billerica, MA, USA) as the enzyme source. This technology utilizes the fluorescence resonance energy transfer ("FRET") process between fluorescein and coumarin. The assay principle is based on the differential sensitivity of phosphorylated and non-phosphorylated peptide to proteolytic cleavage, which precludes the energy transfer process between the two fluorophores attached to both sides of the cleavage site. Hence, phosphorylation by GSK3β will yield a phosphopeptide, which cannot be hydrolyzed by a suitable protease and energy transfer between the two fluorophores will occur. Opposingly, lack of phosphorylation will cause peptide hydrolysis hence lack of energy transfer. The assay is performed in 384-well black plates, in a final volume of 10 μl, with 2 nM enzyme concentration in 50 mM Hepes pH 7.5, 50 mM $MgCl_2$, 1 mM EGTA and 0.05% Brij-35, using 12.5 μM ATP and 2 μM substrate peptide. The latter is a synthetic peptide, provided by Invitrogen under the commercial name "Ser/Thr 9 peptide", and it is based upon the sequence of a GSK3β substrate protein (glycogen synthase I) containing Ser-641. The peptide is labeled at both ends with fluorescein and coumarin. The assay is carried out in the presence of different concentrations of the tested compound, at a final 1% (v/v) DMSO concentration. After a 60 min incubation at room temperature, 5 μl of a commercial protease solution (sold by the same vendor in the assay kit) is added and a subsequent 1h incubation at room temperature is performed, before adding 5 μl of a suitable "stop solution", also provided by the vendor in the kit. After that, fluorescence intensity is recorded, monitoring emission at both 445 and 520 nm, upon excitation at 400 nm. An emission ratio is finally calculated, using the quotient among the emission at 445 nm divided by that at 520 nm.

In the assay plate, several wells are included as a control for full enzyme activity, these wells do not contain any inhibitor or tested substance. Likewise, several wells are also included as a control for lack of enzyme activity, thus these wells do not contain inhibitor nor enzyme. The emission ratio of each tested sample is normalized to that of the control wells, so that for every compound concentration the percentage of inhibition is calculated by using the following equation:

$$\% \text{ Inhibition} = 100 \cdot \frac{E-S}{E-B}$$

wherein "S" is the emission ratio of the sample being considered, "E" is the average emission ratio of wells, including

enzyme in the absence of inhibitors, and "B" is the average emission ratio of wells without enzyme. The inhibition values obtained at every compound concentration are finally used to calculate the EC50 of the tested compound, this parameter being the concentration of the compound which causes 50% of inhibition. For that purpose, the data were fitted to a classical 4-parameters isotherm equation using the nonlinear regression function of GraphPad™ Prism 5.0 (GraphPad Software Inc.). Such equation is described below:

$$\% \text{ Inhibition} = L + \frac{H - L}{1 + \left(\frac{EC50}{C}\right)^n}$$

wherein "L" is the lower asymptote of the theoretical sigmoidal curve, "H" is the higher asymptote, "C" is the concentration of compound and "n" is the Hill coefficient.

In **Table 2,** the EC50 values obtained for some compounds of Formula I are indicated:

<u>**Table 2**</u>

| Compound No. | Structure | GSK3β inhibition average EC50 (μM) |
|---|---|---|
| **Compound** 1 | See table 1 | 0.708 |
| **Compound 2** | See table 1 | 0.066 |
| **Compound 3** | See table 1 | 0.161 |
| **Compound 4** | See table 1 | 0.058 |
| **Compound 5** | See table 1 | 0.125 |
| **Compound 6** | See table 1 | 0.229 |
| **Compound 7** | See table 1 | 0.082 |
| **Compound 8** | See table 1 | 0.097 |
| **Compound 9** | See table 1 | 0.035 |
| **Compound 10** | See table 1 | 0.295 |
| **Compound 11** | See table 1 | 0.104 |
| **Compound 12** | 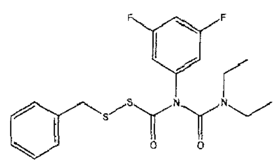 | 8.318 |
| **Compound 13** | 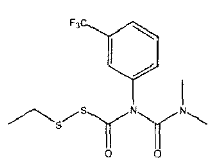 | 0.339 |
| **Compound 15** | 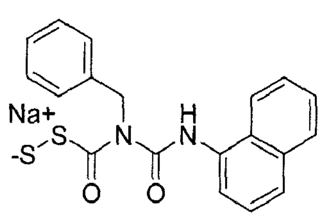 | 1.66 |

**Example 18: Cellular assay**

The effect of the compound in cells is determined by monitoring the level of phosphorylation at the Ser-396 residue of Tau protein in human neuroblastoma SH-SY5Y cells. Cells are grown in 96-well plates: 30,000 cells per well in a volume of 100 μl of 1:1 MEM:Ham's F12 medium including 10% (v/v) fetal bovine serum, 100 U/ml penicillin, 100 μg/ml streptomycin and 1% (v/v) 100-fold concentrated commercial stock of non-essential aminoacids (Gibco, Carlsbad, CA, USA), and incubated at 37°C during 24 h. Then, the corresponding amount of test compound is added to the wells to reach the desired concentration in 1% (v/v) DMSO. The system is incubated at 37°C during 18 h and afterwards cells are harvested, washed with phosphate buffer saline and incubated during 30 min on ice with 120 μl per well of "lysis buffer" (10 mM Tris-HCl pH 7.4, 100 mM NaCl, 1 mM EDTA, 2 mM sodium orthovanadate, 1% (v/v) Triton X-100, 10% (v/v) glycerol, 0.1% (w/v) sodium dodecyl sulfate, 0.5 % (w/v) sodium deoxycholate, 4% (v/v) 25-fold concentrated commercial stock of protease inhibitors (Roche, Basel, Switzerland) and 1 mM phenylmethylsulfonyl fluoride). 60 μl of this mixture is then placed in an empty 96-well plate and the levels of Ser-396 phosphorylation are determined using a commercial ELISA kit (Biosource, Carlsbad, CA, USA), following manufacturer's instructions. The levels of phosphorylation are expressed as percentage values, after normalizing them to those in untreated cells and those values are used to calculate EC50s, as described above for GSK-3.

In **Table 3,** the EC50 values obtained for some compounds of Formula I are indicated:

**Table 3**

| **Compound No.** | **pS*er396-Tau** |
|---|---|
| | **average EC50 (μM)** |
| **Compound 4** | 6.61 |
| **Compound 6** | 6.761 |
| **Compound 10** | 7.08 |

**PHARMACEUTICAL COMPOSITIONS**

In the following, the detailed preparation of some pharmaceutical compositions is described.

**Example 19: Powder for injectable suspension**

**Composition:**

| **INJECTABLE** | **mg/ml** | **%** |
|---|---|---|
| Active ingredient (Compound of Formula I) | 10 | 1 |
| Methylparaben | 1 | 0.1 |
| Prophylparaben | 0.1 | 0.01 |
| Propyleneglycol | 100 | 10 |
| Sodium metabisulfite | 0.25 | 0.025 |
| Sodium chloride | 8.5 | 0.85 |
| Water for injection | csp | |

**Manufacturing Process:**

Powder for suspension

- Fill the active ingredient into vials

Diluent

- Dissolve methylparaben, prophylparaben, sodium metabitsulfite, sodium chloride in propyleneglycol. Mix for a suitable time
- Add water for injection and mix for a suitable time.
- Sterilise by filtration and fill into vials

For final reconstitution before administration, put the diluent solution into the active ingredient vial and shake up to homogeneization.

A list of suitable excipients for injectable suspensions has been detailed above.

**Example 20: Film coated tablet**

**Composition:**

| FILM COATED TABLETS | mg/tablet | % (over FCT) |
|---|---|---|
| **Tablet Core:** | | |
| Active ingredient (Compound of Formula I) | 400 | 55.5 |
| Microcrystalline cellulose | 136 | 18.9 |
| Povidone K-25 | 5 | 0.7 |
| Maize starch | 10 | 1.4 |
| Maize Starch, pregelatinised | 25 | 3.5 |
| Lactose | 100 | 13.9 |
| Sodium croscarmellose | 7 | 1.0 |
| Talc | 12 | 1.7 |
| Magnesium stearate | 5 | 0.7 |
| Total (core) | 700 | |
| **Film coating**: | | |
| Hypromellose | 8 | 1.1 |
| Titanium dioxide | 5 | 0.7 |
| Macrogol/PEG 4000 | 3 | 0.4 |
| Lactose | 4 | 0.6 |
| Total (tablet) | 720 | |

**Manufacturing Process:**

- Prepare granulation solution dissolving Povidone K-25 in water
- Mix the active ingredient, maize starch, maize starch pregelatinised and microcrystalline cellulose.
- Granulate with granulation solution
- Drying

- Sieve the dried granules through a suitable mesh size
- Add lactose, sodium croscarmellose and talc
- Mix for a suitable time
- Add magnesium stearate
- Mix for a suitable time
- Once final blend is finished, then is ready for tabletting.
- Tablet compression
- Film coating

A list of suitable excipients for coated tablets has been detailed above.

**Example 21: Powder for oral solution (POS) in sachet**

**Composition:**

| POS-SACHETS | mg/sachet | % |
|---|---|---|
| Active ingredient (Compound of Formula I) | 500 | 9.9% |
| Citric acid | 150 | 3.0% |
| Sodium citrate | 100 | 2.0% |
| Manitol | 2500 | 49.5% |
| Sorbitol | 1500 | 29.7% |
| Sucralose | 150 | 3.0% |
| Aerosil 200 | 5 | 0.1% |
| Lemon flavour | 75 | 1.5% |
| Cola flavour | 75 | 1.5% |
| Total | 5,055 | |

**Manufacturing Process:**

- Pass all the components through a suitable mesh size
- Mix the components into a suitable mixer
- Discharge final blend into containers
- Dosage the blend into sachets

A list of suitable excipients for powders for oral solution has been detailed above.

**Example 22: Syrup**

**Composition:**

| SYRUP | mg/ml | % |
|---|---|---|
| Active ingredient (Compound of Formula I) | 5 | 0.5 |
| Propyleneglycol | 50 | 5 |
| Ethanol | 10 | 1 |
| Sorbitol | 250 | 25 |
| Sodium benzoate | 1.5 | 0.15 |
| Citric acid | 20 | 2 |
| Sodium citrate | 15 | 1.5 |
| Vainille flavour | 1.2 | 0.12 |
| Tartrazine | 30 | 3 |
| Purified water | csp | |
| Total | 380 | |

**Manufacturing Process:**

- Put propyleneglycol and ethanol in a suitable container
- Add sodium benzoate up to total dissolution
- Add citric acid and sodium citrate and mix up to total dissolution
- Add active ingredient and mix up to homogeneization
- Add sorbitol and mix up to homogeneization
- Add purified water and mix up to homogeneization
- Add vainille flavour and tartrazine and mix up to homogeneization
- Once the syrup bulk is finished, it is ready for dosing into glass or plastic bottles.

A list of suitable excipients for syrups has been detailed above.

**Example 23: Capsules**

**Composition:**

| CAPSULES | mg/capsule | % |
|---|---|---|
| Active ingredient (Compound of Formula I) | 400 | 66.7 |
| Microcrystalline cellulose | 85 | 14.2 |
| Calcium phosphate | 100 | 16.7 |
| Talc | 10 | 1.7 |
| Magnesium stearate | 5 | 0.8 |
| Total | 600 | |

**Manufacturing Process:**

- Pass all the components through a suitable mesh size
- Put active ingredient, cellulose microcrystalline, calcium phosphate and talc into a suitable mixer.
- Mix for a suitable time
- Add magnesium stearate
- Mix for a suitable time
- Once the final blend is finished, it is ready to be dosed into gelatin capsules (suitable size)

A list of suitable excipients for capsules has been detailed above.

**Example 24: Gastrorresistant capsules**

**Composition:**

| GASTRORRESISTANT CAPSULES (pellets) | mg/capsule | % |
|---|---|---|
| Active ingredient (Compound of Formula I) | 40 | 10 |
| Microcrystalline cellulose spheres | 260 | 65 |
| Poloxamer | 15 | 3.8 |
| Copolymer methacrylic acid | 80 | 20.0 |
| Pftalate dibutyl | 2 | 0.5 |
| Erythrosine | 3 | 0.8 |
| Isopropyl alcohol | eliminated during process | |
| Acetone | eliminated during process | |
| Total | 400 | |

**Manufacturing Process:**

- Dissolve erythrosine, phthalate dibutyl and copolymer methacrylic
- acid in acetone + isopropylalcohol.
- Add active ingredient and the poloxamer and dissolve it in the previous solution.
- Put the microcrystalline cellulose spheres into a fluid bed dryer.
- Spray the coating solution over on the cellulose spheres.
- Once the coating solution has been totally sprayed, dry the granules.
- Once dried, discharge into a suitable container

- Fill gelatine capsules with the coated spheres

A list of suitable excipients for gastrorresistant capsules has been detailed above.

## Claims

1. A compound of Formula (I)

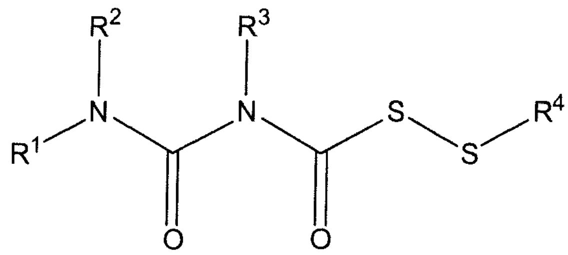

**Formula (I)**

wherein
$R^1$ is selected from optionally substituted $C_6$-$C_{14}$ aryl; $C_7$-$C_{20}$ arylalky; optionally substituted $C_1$-$C_6$ linear or branched alkyl; or a benzo-fused heterocyclic ring system; $R^2$ is selected from hydrogen; or $C_1$-$C_6$ linear or branched alkyl;
$R^3$ is selected from $C_7$-$C_{20}$ arylalky; or optionally substituted $C_1$-$C_6$ linear or branched alkyl; optionally substituted $C_6$-$C_{10}$ aryl;
$R^4$ is selected from hydrogen, $C_3$-$C_6$ cycloalkyl; $C_7$-$C_{20}$ arylalky; $C_2$-$C_6$ alkenyl; optionally substituted $C_1$-$C_6$ linear or branched alkyl;
or a salt, solvate or prodrug thereof;
with the proviso that the compound is not Carbamo(dithioperoxoic) acid, [(dimethylamino)carbonyl][3-(trifluoromethyl)phenyl]-, ethyl ester.

2. A compound according to claim 1, wherein
$R^1$ is selected from $C_6$-$C_{14}$ aryl, optionally substituted by phenoxy; $C_7$-$C_{20}$ arylalky; $C_1$-$C_6$ linear or branched alkyl, optionally substituted by acetic acid alkyl ester; or a benzo-fused heterocyclic ring system;
$R^2$ is selected from hydrogen; or $C_1$-$C_6$ linear or branched alkyl;
$R^3$ is selected from $C_7$-$C_{20}$ arylalky; or $C_1$-$C_6$ linear or branched alkyl, optionally substituted by acetic acid alkyl ester; $C_6$-$C_{14}$ aryl, optionally substituted by halogen or $C_1$-$C_6$ haloalkyl;
$R^4$ is selected from $C_3$-$C_6$ cycloalkyl; $C_7$-$C_{20}$ arylalky; $C_2$-$C_6$ alkenyl; $C_1$-$C_6$ linear or branched alkyl, optionally substituted by acetic acid alkyl ester or N-acetamide;
or a salt, solvate or prodrug thereof;

3. A compound according to claim 1, wherein
$R^1$ is selected from napthyl, phenoxy-phenyl, arylmethyl, benzo[1,3]dioxol, acetic acid ethyl ester, ethyl, or methyl;
$R^2$ is selected from hydrogen, methyl, or ethyl;
$R^3$ is selected from phenylmethyl, acetic acid ethyl ester, or phenyl, optionally substituted by halogen or $C_1$-$C_6$ halomethyl;
$R^4$ is selected from propenyl, $C_2$-$C_5$ linear or branched alkyl, cyclopentyl, phenylethyl, phenylmethyl;
or a salt, solvate or prodrug thereof.

4. A compound according to claim 1, selected from:

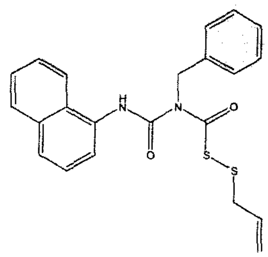

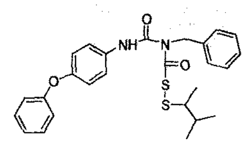

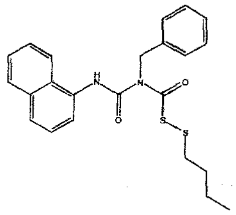

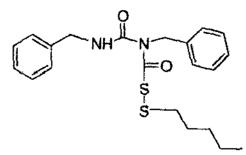

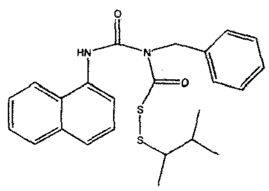

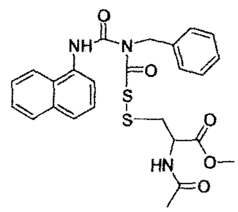

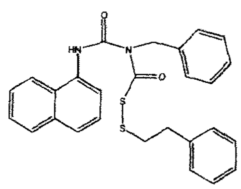

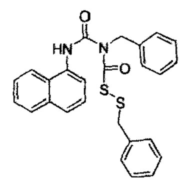

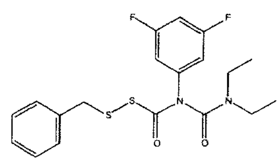

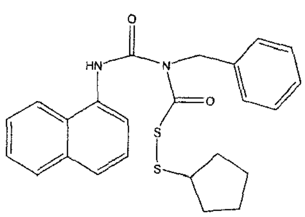

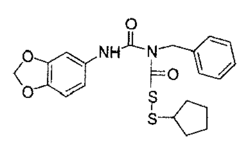

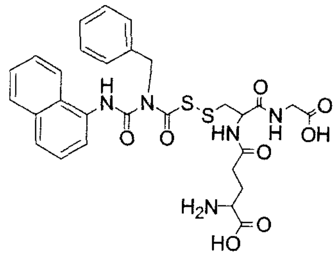

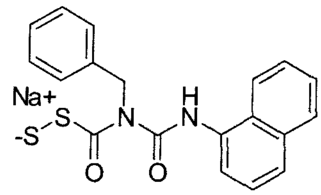

**5.** A compound of Formula (I)

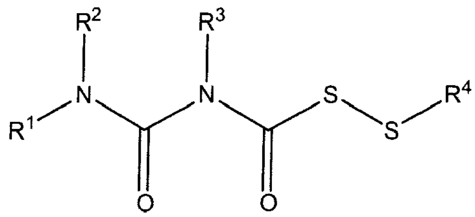

**Formula (I)**

wherein
$R^1$ is selected from optionally substituted $C_6$-$C_{14}$ aryl; $C_7$-$C_{20}$ arylalky; optionally substituted $C_1$-$C_6$ linear or branched alkyl; or a benzo-fused heterocyclic ring system; $R^2$ is selected from hydrogen; or $C_1$-$C_6$ linear or branched alkyl;
$R^3$ is selected from $C_7$-$C_{20}$ arylalky; or optionally substituted $C_1$-$C_6$ linear or branched alkyl; optionally substituted $C_6$-$C_{14}$ aryl;
$R^4$ is selected from hydrogen, $C_3$-$C_6$ cycloalkyl; $C_7$-$C_{20}$ arylalky; $C_2$-$C_6$ alkenyl; optionally substituted $C_1$-$C_6$ linear or branched alkyl;
or a salt, solvate or prodrug thereof; for use as a medicament.

**6.** A compound according to claim 5, wherein
$R^1$ is selected from $C_6$-$C_{14}$ aryl, optionally substituted by phenoxy; $C_7$-$C_{20}$ arylalky; $C_1$-$C_6$ linear or branched alkyl, optionally substituted by acetic acid alkyl ester; or a benzo-fused heterocyclic ring system;
$R^2$ is selected from hydrogen; or $C_1$-$C_6$ linear or branched alkyl;
$R^3$ is selected from $C_7$-$C_{20}$ arylalky; or $C_1$-$C_6$ linear or branched alkyl, optionally substituted by acetic acid alkyl ester; $C_6$-$C_{14}$ aryl, optionally substituted by halogen or $C_1$-$C_6$ haloalkyl;
$R^4$ is selected from $C_3$-$C_6$ cycloalkyl; $C_7$-$C_{20}$ arylalky; $C_2$-$C_6$ alkenyl; $C_1$-$C_6$ linear or branched alkyl, optionally substituted by acetic acid alkyl ester or N-acetamide;
or a salt, solvate or prodrug thereof;

**7.** A compound according to claim 5, wherein
$R^1$ is selected from napthyl, phenoxy-phenyl, arylmethyl, benzo[1,3]dioxol, acetic acid ethyl ester, ethyl, or methyl;
$R^2$ is selected from hydrogen, methyl, or ethyl;
$R^3$ is selected from phenylmethyl, acetic acid ethyl ester, or phenyl, optionally substituted by halogen or halomethyl;
$R^4$ is selected from propenyl, $C_2$-$C_5$ linear or branched alkyl, cyclopentyl, phenylethyl, phenylmethyl;
or a salt, solvate or prodrug thereof.

**8.** The compound according to claim 5, selected from:

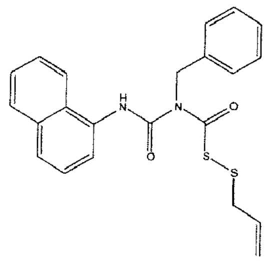

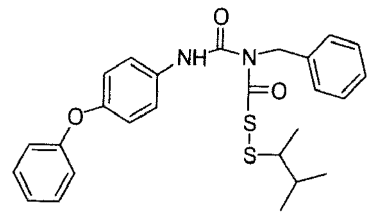

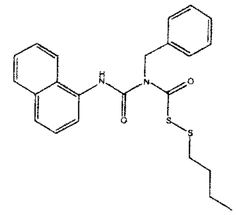

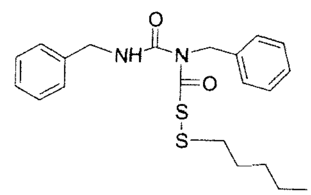

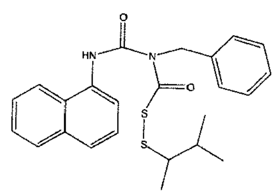

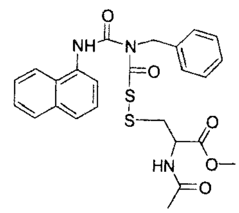

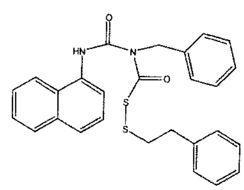

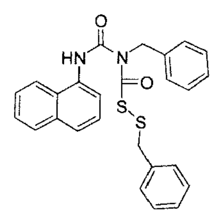

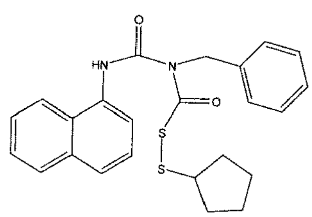

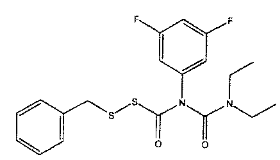

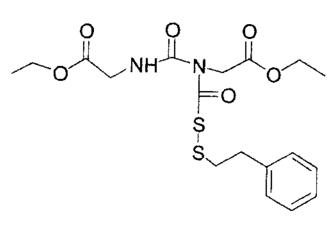

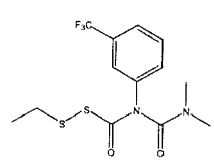

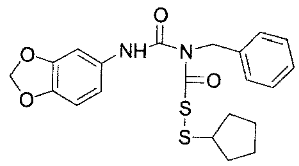

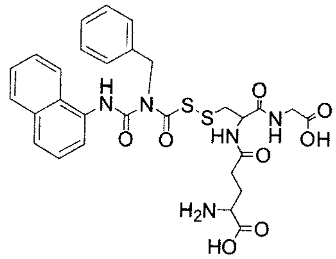

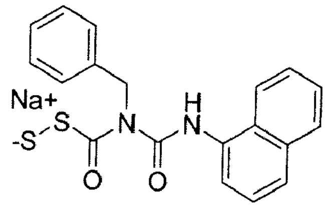

9. A pharmaceutical composition comprising a compound of Formula (I) as defined in any one of claims 5 to 8, or a salt, solvate or prodrug thereof, and at least one pharmaceutically acceptable carrier, adjuvant, and/or vehicle.

10. A compound of Formula (I) as defined in any one of claims 5 to 8, or a salt, solvate or prodrug thereof, for use in the treatment of a cognitive, neurodegenerative or neurological disease or condition.

11. A compound according to claim 10, wherein the cognitive, neurodegenerative or neurological disease or condition is selected from Alzheimer's disease, Parkinson's disease, frontotemporal dementia, Pick disease, progressive supranuclear palsy, subacute sclerosing panencephalitis, postencephalitic parkinsonism, dementia pugilistica, guam parkinsonism-dementia complex, corticobasal degeneration, argyrophilic grain disease, familial frontotemporal dementia and parkinsonism linked to chromosome 17 due to mutations in the tau gene (FTDP-17-tau), AIDS associated dementia, Huntington's disease, Lewy body disease, bipolar disorder, depression, schizophrenia, epilepsy, mood disorders, autism, attention deficit hyperactivity disorder, Down's syndrome, ischemia/reperfusion, shock, brain injury, multiple sclerosis, autoimmune and inflammatory diseases afflicting the CNS, spinocerebellar ataxia type 1, cerebral bleeding due to solitary cerebral amyloid angiopathy, amyotrophic lateral sclerosis, mild cognitive impairment, Niemann-Pick disease, frontotemporal lobar degeneration, including progressive apraxia and progressive non-fluent aphasia, multiple system tauopathy with presenile dementia, chronic traumatic encephalopathy (CTE) and head trauma, pallido-ponto-nigral degeneration, prion disease, Gerstman-Straussler-Scheinker disease, Hallervordern-Spatz disease, and myotonic dystrophy.

12. A compound of Formula (I) as defined in any one of claims 5 to 8, or a salt, solvate or prodrug thereof, for use in the treatment of a disease or condition selected from inflammatory and autoimmune diseases including rheumatoid arthritis, inflammatory bowel disease and psoriasis, arthritis, peritonitis, systemic inflammation, renal dysfunction and hepatotoxicity in endotoxemia, asthma, sepsis, colitis, inflammation-induced organ injury caused by hemorrhage and resuscitation, inflammatory injury in chronic renal allograft disease and lupus.

13. Use of a compound of Formula (I) as defined in any one of claims 5 to 8, or a salt, solvate or prodrug thereof, as a reactive in an *in vitro* biological assay requiring inhibition of GSK-3.

14. Process for obtaining a compound of Formula (II):

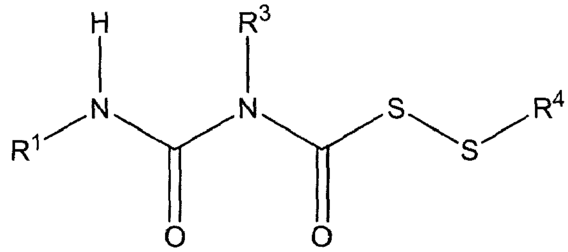

**Formula (II)**

wherein $R^1$, $R^3$ and $R^4$ are as defined in claims 1 to 4, comprising the steps of:

a) dissolving in a suitable solvent a [1,2,4]thiadiazolidine-3,5-dione of formula

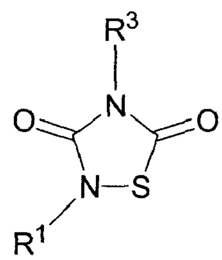

wherein $R^1$ and $R^3$ have the same meaning as in the compound of Formula (II);
adding a thiol derivative according to the desired final product: $R^4$-SH, wherein $R^4$ has the same meaning as in the compound of Formula (II);
stirring; and
b) isolating said compound of formula (II) by filtration, by preparative HPLC or with a Flash purification system.

Europäisches Patentamt
European Patent Office
Office européen des brevets

# EUROPEAN SEARCH REPORT

Application Number
EP 11 38 2164

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | US 4 119 432 A (GAUGHAN EDMUND J) 10 October 1978 (1978-10-10) * column 1; example 1 * ----- | 1 | INV. C07C333/10 A61K31/325 |
| A,D | BHAT R ET AL: "Structural Insights and Biological Effects of Glycogen Synthase Kinase 3-specific Inhibitor AR-A014418", JOURNAL OF BIOLOGICAL CHEMISTRY, THE AMERICAN SOCIETY OF BIOLOGICAL CHEMISTS, INC, US, vol. 278, no. 46, 14 November 2003 (2003-11-14), pages 45937-45945, XP002999443, ISSN: 0021-9258, DOI: 10.1074/JBC.M306268200 * the whole document * ----- | 1-14 | |
| A,D | MARTINEZ A ET AL: "First Non-ATP Competitive Glycogen Synthase Kinase 3.beta. (GSK-3.beta.) Inhibitors: Thiadiazolidinones (TDZD) as Potential Drugs for the Treatment of Alzheimer's Disease", JOURNAL OF MEDICINAL CHEMISTRY, AMERICAN CHEMICAL SOCIETY, US, vol. 45, no. 6, 1 January 2002 (2002-01-01), pages 1292-1299, XP002292084, ISSN: 0022-2623, DOI: 10.1021/JM011020U * the whole document * ----- | 1-14 | TECHNICAL FIELDS SEARCHED (IPC) C07C |

The present search report has been drawn up for all claims

1

| Place of search | Date of completion of the search | Examiner |
|---|---|---|
| Munich | 28 September 2011 | Bedel, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 38 2164

This annex lists the patent family members relating to the patent documents cited in the above-mentioned European search report. The members are as contained in the European Patent Office EDP file on
The European Patent Office is in no way liable for these particulars which are merely given for the purpose of information.

28-09-2011

| Patent document cited in search report | Publication date | Patent family member(s) | Publication date |
|---|---|---|---|
| US 4119432 A | 10-10-1978 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4119432 A
- WO 0185685 A
- WO 0597117 A
- DE 1219925


### Non-patent literature cited in the description


- *Chemistry & Biology,* 2000, vol. 7 (10), 793-803
- **COGHLAN et al.** *Curr. Opinion Genetics Dev.,* 2000, vol. 10 (5), 508-514
- *Physiol. Rev.,* 2004, vol. 84, 361-84
- *Circ Res.,* 2009, vol. 104 (11), 1240-52
- **JUHASZOVA M. et al.** *Circ J.,* 2009, vol. 73 (7), 1184-92
- **ELDAR-FINKELMAN, H.** Glycogen synthase kinase 3: an emerging therapeutic target. *Trends. Mol. Med.,* 2002, vol. 8, 126-32
- **ASENJO, A.** Residues in human respiratory syncytial virus P protein that are essential for its activity on RNA viral synthesis. *Virus Res.,* 2008, vol. 132, 160-73
- **HERNÁNDEZ F.** The role of GSK3 in Alzheimer disease. *Brain Res Bull.,* 2009, vol. 80 (4-5), 248-50
- **MASAHIRO N. ; HIDEAKI H.** Glycogen synthase kinase-3beta is associated with Parkinson's disease. *Neuroscience Letters,* 2009, vol. 449 (2), 103-107
- **SCHAFFER, B.** Association of GSK3B with Alzheimer disease and frontotemporal dementia. *Arch. Neurol.,* 2008, vol. 65, 1368-74
- *Brain Research Reviews,* 2000, vol. 33, 95-130
- **DEWHURST S.** Glycogen synthase kinase 3 beta (GSK-3 beta) as a therapeutic target in neuroAIDS. *J Neuroimmune Pharmacol.,* 2007, vol. 2 (1), 93-96
- **CARMICHAEL J.** Glycogen synthase kinase-3beta inhibitors prevent cellular polyglutamine toxicity caused by the Huntington's disease mutation. *J Biol Chem.,* 2002, vol. 277 (37), 33791-8
- **TANJI K.** Glycogen synthase kinase-3beta phosphorylates synphilin-1 in vitro. *Neuropathology,* 2003, vol. 23 (3), 199-202
- **ROEW M.K.** GSK-3 is a viable potential target for therapeutic intervention in bipolar disorder. *Neurosci Biobehav Rev.,* 2007, vol. 31 (6), 920-931
- **LI X.** Glycogen Synthase Kinase-3β, mood stabilizers, and neuroprotection. *Bipolar Disord.,* 2002, vol. 4 (2), 137-144
- **WADA A.** Lithium and neuropsychiatric therapeutics: neuroplasticity via glycogen synthase kinase-3beta, beta-catenin, and neurotrophin cascades. *J Pharmacol Sci.,* 2009, vol. 110 (1), 14-28
- **KOROS E. ; DOMER-CIOSSEK C.** The role of glycogen synthase kinase-3beta in schizophrenia. *Drug News Perspect.,* 2007, vol. 20 (7), 437-45
- **LOVESTONE S.** Schizophrenia as a GSK-3 dysregulation disorder. *Trends Neurosci.,* 2007, vol. 30 (4), 142-9
- **CHEN G.** The mood-stabilizing agent valproate inhibits the activity of glycogen synthase kinase-3. *J Neurochem.,* 1999, vol. 72 (3), 1327-30
- **JOPE R. S. ; ROH M.S.** Glycogen synthase kinase-3 (GSK3) in psychiatric diseases and therapeutic interventions. *Curr Drug Targets.,* 2006, vol. 7 (11), 1421-34
- **BEAULIEU J.M.** Role of GSK3 beta in behavioral abnormalities induced by serotonin deficiency. *Proc Natl Acad Sci U S A.,* 2008, vol. 105 (4), 1333-8
- **BEAULIEU J.M.** Lithium antagonizes dopamine-dependent behaviors mediated by an AKT/glycogen synthase kinase 3 signaling cascade. *Proc Natl Acad Sci U S A.,* 2004, vol. 101 (14), 5099-104
- **LIU F.** Overexpression of Dyrk1A contributes to neurofibrillary degeneration in Down syndrome. *FASEB J.,* 2008, vol. 22 (9), 3224-33
- **DUGO L.** Glycogen synthase kinase 3beta as a target for the therapy of shock and inflammation. *Shock.,* 2007, vol. 27 (2), 113-23
- **SASAKI C.** Different expression of glycogen synthase kinase-3beta between young and old rat brains after transient middle cerebral artery occlusion. *Neurol Res.,* 2001, vol. 23 (6), 588-92
- **BEUREL E.** Innate and adaptive immune responses regulated by glycogen synthase kinase-3 (GSK3). *Trends Immunol.,* 2010, vol. 31 (1), 24-31

- **DE SARNO P.** Lithium prevents and ameliorates experimental autoimmune encephalomyelitis. *J. Immunol.,* 2008, vol. 181 (1), 338-45
- **WATASE K.** Lithium therapy improves neurological function and hippocampal dendritic arborization in a spinocerebellar ataxia type 1 mouse model. *PLoS Med,* 2007, vol. 4 (5), 836-847
- **HAN F.** Accumulation of beta-amyloid in the brain microvessels accompanies increased hyperphosphorylated tau proteins following microsphere embolism in aged rats. *Neuroscience.,* 2008, vol. 153, 414-27
- **YANG W.** Upregulation of GSK3β expression in frontal and temporal cortex in ALS with cognitive impairment (ALSci). *Brain Res.,* 2008, vol. 1196, 131-139
- **DILL, J.** Inactivation of glycogen synthase kinase 3 promotes axonal growth and recovery in the CNS. *J. Neurosci.,* 2008, vol. 28, 8914-28
- **COHEN P.** The role of protein phosphorylation in human health and disease. *Eur J Biochem.,* 2001, vol. 268 (19), 5001-10
- **CUZZOCREA, S.** Glycogen synthase kinase-3b inhibition attenuates the degree of arthritis caused by type II collagen in the mouse. *Clin. Immunol.,* 2006, vol. 120, 57-67
- **HU, X.** IFN-g suppresses IL-10 production and synergizes with TLR2 by regulating GSK3 and CREB/AP-1 proteins. *Immunity,* 2006, vol. 24, 563-574
- **DUGO, L.** GSK-3b inhibitors attenuate the organ injury/dysfunction caused by endotoxemia in the rat. *Crit. Care Med.,* 2005, vol. 33, 1903-1912
- **BENTLEY J.K.** Airway smooth muscle hyperplasia and hypertrophy correlate with glycogen synthase kinase-3(beta) phosphorylation in a mouse model of asthma. *Am J Physiol Lung Cell Mol Physiol.,* 2009, vol. 296 (2), L176-84
- **EVENSON, A.R.** GSK-3b inhibitors reduce protein degradation in muscles from septic rats and in dexamethasone treated myotubes. Int. *J. Biochem. Cell. Biol.,* 2005, vol. 37, 2226-2238
- **WHITTLE, B.J.** Reduction of experimental colitis in the rat by inhibitors of glycogen synthase kinase-3b. *Br. J. Pharmacol.,* 2006, vol. 147, 575-582
- **DUGO, L.** Glycogen synthase kinase-3b inhibitors protect against the organ injury and dysfunction caused by hemorrhage and resuscitation. *Shock,* 2006, vol. 25, 485-491
- **GONG R.** Glycogen synthase kinase 3beta: a novel marker and modulator of inflammatory injury in chronic renal allograft disease. *Am J Transplant.,* 2008, vol. 8 (9), 1852-63
- **LENZ, S.P.** Lithium chloride enhances survival of NZBIW lupus mice: influence of melatonin and timing of treatment. *Int. J. Immunopharmacol.,* 1995, vol. 17, 581-592
- **HARDT, S.E. ; SADOSHIMA, J.** Glycogen synthase kinase-3beta: a novel regulator of cardiac hypertrophy and development. *Circ. Res.,* 2002, vol. 90, 1055-63
- **BOWES A.J.** Valproate attenuates accelerated atherosclerosis in hyperglycemic apoE-deficient mice: evidence in support of a role for endoplasmic reticulum stress and glycogen synthase kinase-3 in lesion development and hepatic steatosis. *Am J Pathol.,* 2009, vol. 174 (1), 330-42
- **BADORFF C.** Fas receptor signaling inhibits glycogen synthase kinase 3β and induces cardiac hypertrophy following pressure overload. *J. Clin. Invest.,* 2002, vol. 109 (3), 373-381
- **MA X.** Delayed Re-endothelialization with Rapamycin-coated Stents is Rescued by the Addition of a Glycogen Synthase Kinase 3 Beta Inhibitor. *Cardiovasc Res.,* 2010
- **GALLICCHIO, V. S.** Lithium and the Cell. Academic, 1991, 185-198
- **WAGMAN A.S.** Discovery and development of GSK3 inhibitors for the treatment of type 2 diabetes. *Curr Pharm Des.,* 2004, vol. 10 (10), 1105-37
- **LYUBIMOVA A.** Neural Wiskott-Aldrich syndrome protein modulates Wnt signaling and is required for hair follicle cycling in mice. *J Clin Invest.,* 2010, vol. 120 (2), 446-56
- **WU C.H.** Glycogen synthase kinase-3 regulates the phosphorylation of severe acute respiratory syndrome corona virus nucleocapsid protein and viral replication. *J Biol Chem.,* 2009, vol. 284 (8), 5229-39
- **XU C.M.** Glycogen synthase kinase 3beta in the nucleus accumbens core mediates cocaine-induced behavioral sensitization. *J Neurochem.,* 2009, vol. 111 (6), 1357-68
- **WANG F.S.** Inhibition of glycogen synthase kinase-3beta attenuates glucocorticoid-induced bone loss. *Life Sci.,* 2009, vol. 85 (19-20), 685-92
- **YUSKAITIS C.J.** Lithium ameliorates altered glycogen synthase kinase-3 and behavior in a mouse model of fragile X syndrome. *Biochem Pharmacol.,* 2010, vol. 79 (4), 632-46
- **WANG W.H.** Increased expression of the WNT antagonist sFRP-1 in glaucoma elevates intraocular pressure. *J Clin Invest.,* 2008, vol. 118 (3), 1056-64
- **COHEN, P. ; GOEDERT, M.** GSK3 inhibitors: development end therapeutic potential. *Nature Reviews,* 2004, vol. 3, 479-487
- **HERNÁNDEZ, F.** GSK3 Inhibitors and Disease. *Mini-Reviews in Medicinal Chemistry,* 2009, vol. 9 (9), 1024-1029
- **MEDINA, M. ; CASTRO, A.** Glycogen synthase kinase-3 (GSK-3) inhibitors reach the clinic. *Curr. Opin. Drug Discov. Develop.,* 2008, vol. 11 (4), 533-543

- Glycogen Synthase Kinase 3 (GSK-3) and its inhibitors. John Wiley & Sons, Inc, 2006
- **LECLERC, S.** Indirubins inhibit glycogen synthase kinase-3 beta and CDK5/p25, two protein kinases involved in abnormal tau phosphorylation in Alzheimer's disease. A property common to most cyclin-dependent kinase inhibitors?. *J. Biol. Chem.,* 2001, vol. 276, 251-60
- **SMITH, D.** 3-Anilino- 4-arylmaleimides: potent and selective inhibitors of glycogen synthase kinase-3 (GSK-3). *Bioorg. Med. Chem. Lett.,* 2001, vol. 11, 635-9
- **WITHERINGTON, J.** 5-arylpyrazolo[3,4-b]pyridazines: potent inhibitors of glycogen synthase kinase-3 (GSK-3). *Bioorg. Med. Chem. Lett.,* 2003, vol. 13, 1581-4
- **LEOST, M.** Paullones are potent inhibitors of glycogen synthase kinase-3beta and cyclin-dependent kinase 5/p25. *Eur. J. Biochem.,* 2000, vol. 267, 5983-94
- **BHAT, R.** Structural insights and biological effects of glycogen synthase kinase 3-specific inhibitor AR-A014418. *J. Biol. Chem.,* 2003, vol. 278, 45937-45
- **MARTINEZ, A.** First non-ATP competitive glycogen synthase kinase 3 beta (GSK-3beta) inhibitors: thiadiazolidinones (TDZD) as potential drugs for the treatment of Alzheimer's disease. *J. Med. Chem.,* 2002, vol. 45, 1292-9
- *Acta Neuropathol.,* 2009, vol. 118 (1), 53-69
- **IQBAL, K. ; BUÉE, L. et al.** Tau protein isoforms, phosphorylation and role in neurodegenerative disorders. *Brain Research Reviews,* 2000, vol. 33, 95-130
- **IQBAL, K.** Mechanisms of tau-induced neurodegeneration. *Acta Neuropathol.,* 2009, vol. 118 (1), 53-69
- **COMPTA, Y.** Cerebrospinal tau, phospho-tau, and beta-amyloid and neuropsychological functions in Parkinson's disease. *Mov Disord.,* 2009, vol. 24 (15), 2203-10
- **BREW, B.J.** CSF amyloid beta42 and tau levels correlate with AIDS dementia complex. *Neurology,* 2005, vol. 65 (9), 1490-2
- **MANN, D.M.A.** Alzheimer's presenile dementia, senile dementia of Alzheimer type and Down's syndrome in middle age form an age related continuum of pathological changes. *Neuropathol. Appl. Neurobiol.,* 1984, vol. 10, 185-207
- **MITCHELL , A.J.** CSF phosphorylated tau in the diagnosis and prognosis of mild cognitive impairment and Alzheimer's disease: a meta-analysis of 51 studies. *J Neurol Neurosurg Psychiatry,* 2009, vol. 80 (9), 966-75
- **FREDERICK, J.** Pick disease. A Brief Overview. *Arch. Pathol. Lab. Med.,* 2006, vol. 130, 1063-1066
- **HANSON, J.C. ; LIPPA, C.F.** Lewy body dementia. *Int Rev Neurobiol.,* 2009, vol. 84, 215-28
- **LOVE, S.** Neurofibrillary tangles in Niemann-Pick disease type C. *Brain,* 1985, vol. 118, 119-129
- **FERRER, I.** Argyrophilic grain disease. *Brain.,* 2008, vol. 131 (6), 1416-32
- **SNOWDEN, J.** Frontotemporal lobar degeneration: clinical and pathological relationships. *Acta Neuropathol.,* 2007, vol. 114 (1), 31-8
- **HUTTON, M.** Association of missense and 50-splice-site mutations in tau with the inherited dementia FTDP-17. *Nature,* 1998, vol. 393, 702-705
- **SPINA, S.** The tauopathy associated with mutation +3 in intron 10 of Tau: characterization of the MSTD family. *Brain,* 2008, vol. 131 (1), 72-89
- **HOF, P.R.** Differential distribution of neurofibrillary tangles in the cerebral cortex of dementia pugilistica and Alzheimer's disease cases. *Acta Neuropathol.,* 1992, vol. 85, 23-30
- **MCKEE, A. C.** Chronic traumatic encephalopathy in athletes: progressive tauopathy after repetitive head injury. *J Neuropathol Exp Neurol.,* 2009, vol. 68 (7), 709-35
- **WILLIAMS, D.R. ; LEES, A.J.** Progressive supranuclear palsy: clinicopathological concepts and diagnostic challenges. *Lancet Neurol.,* 2009, vol. 8 (3), 270-9
- **JELLINGER, K.A.** Absence of alpha-synuclein pathology in postencephalitic parkinsonism. *Acta Neuropathol.,* 2009, vol. 118 (3), 371-9
- **YUKSEL, D.** Tau proteins in the cerebrospinal fluid of patients with subacute sclerosing panencephalitis. *Brain Dev.,* 2010, vol. 32 (6), 467-71
- **HIRANO, A.** Amyotrophic lateral sclerosis and Parkinsonism-dementia complex on Guam. Further pathologic studies. *Arch. Neurol.,* 1996, vol. 15, 35-51
- **FELICIAN, O.** Corticobasal degeneration: clinical and neuropsychological profile. *Psychol NeuroPsychiatr Vieil,* 2009, vol. 7 (2), 91-100
- **DELISLE, M.B.** Neurodegenerative disease associated with a mutation of codon 279 (N279K) in exon 10 of Tau protein. *Bull Acad Natl Med.,* 2000, vol. 184 (4), 799-809
- **ASUNI, A.A.** Change in tau phosphorylation associated with neurodegeneration in the ME7 model of prion disease. *Biochem Soc Trans.,* 2010, vol. 38 (2), 545-51
- **GHETTI, B.** Gerstmann-Sträussler-Scheinker disease and the Indiana kindred. *Brain Pathol.,* 1995, vol. 5, 61-75
- **SAITO, Y.** Widespread expression of alpha-synuclein and tau immunoreactivity in Hallervorden-Spatz syndrome with protracted clinical course. *J Neurol Sci.,* 2010, vol. 177 (1), 48-59

- **KIUCHI, A.** Presenile appearance of abundant Alzheimer's neurofibrillary tangles without senile plaques in the brain in myotonic dystrophy. *Acta Neuropathol.,* 1991, vol. 82, 1-5
- **DEUTSCH, S.I.** Dysregulation of tau phosphorylation is a hypothesized point of convergence in the pathogenesis of Alzheimer's disease, frontotemporal dementia and schizophrenia with therapeutic implications. *Prog Neuropsychopharmacol Biol Psychiatry.,* 2006, vol. 30 (8), 1369-80
- **ANDERSON, J.M.** Abnormal tau phosphorylation in primary progressive multiple sclerosis. *Acta Neuropathol.,* 2010, vol. 119 (5), 591-600
- **BALL, M.J.** Neuronal loss, neurofibrillary tangles and granulovacuolar degeneration in the hippocampus with ageing and dementia. A quantitative study. *Acta Neuropathol.,* 1977, vol. 37, 111-118
- **TOMLINSON, B.E.** Observations on the brains of non-demented old people. *J. Neurol. Sci.,* 1968, vol. 7, 331-356
- **GASPARINI, L.** Tau inclusions in retinal ganglion cells of human P301 S tau transgenic mice: Effects on axonal viability. *Neurobiol Aging.,* 07 April 2009
- **BUÉE, L.** Tau protein isoforms, phosphorylation and role in neurodegenerative disorders. *Brain Research Reviews,* 2000, vol. 33, 95-130
- **LUDOLPH, A.C.** Tauopathies with parkinsonism: clinical spectrum, neuropathologic basis, biological markers, and treatment options. *Eur J Neurol.,* 2009, vol. 16 (3), 297-309
- **KROGSGAARD-LARSEN et al.** Textbook of Drug design and Discovery. Taylor & Francis, April 2002
- Stedman's Medical Spellchecker. Lippincott Williams & Wilkins, 2006
- **MARTINEZ, A.** First Non-ATP Competitive Glycogen Synthase Kinase 3 $\alpha$ (GSK-3$\alpha$) Inhibitors: Thiadiazolidinones (TDZD) as Potential Drugs for the Treatment of Alzheimer's Disease. *J. Med. Chem.,* 2002, vol. 45, 1292-1299